# EUROPÄISCHE PATENTSCHRIFT

(11) **EP 1 966 206 B1**
(45) Veröffentlichungstag und Bekanntmachung des Hinweises auf die Patenterteilung: **09.03.2016**
(21) Anmeldenummer: 06841589.2
(22) Anmeldetag: 22.12.2006
(51) Int. Cl.: C07D 471/06, C07D 471/22, C07D 493/06, H01L 51/00

(54) **NAPHTHALINTETRACARBONSÄUREDERIVATE UND DEREN VERWENDUNG ALS HALBLEITER**
NAPHTHALENETETRACARBOXYLIC ACID DERIVATIVES AND THEIR USE AS SEMICONDUCTORS
DERIVE DE L ACIDE NAPHTALINE TETRACARBOXYLIQUE ET SON UTILISATION EN TANT QUE SEMI-CONDUCTEUR

(30) Priorität: 23.12.2005 DE 102005061997
(43) Veröffentlichungstag der Anmeldung: 10.09.2008
(60) Teilanmeldung: 12170817.6
(73) Patentinhaber: BASF SE, 67056 Ludwigshafen (DE)
(72) Erfinder: KÖNEMANN, Martin, 68163 Mannheim (DE)
(86) Internationale Anmeldenummer: PCT/EP2006/070143
(87) Internationale Veröffentlichungsnummer: WO 2007/074137

(56) Entgegenhaltungen:
- DE-A1- 3 703 131
- DE-A1- 10 148 172
- US-A1- 2005 176 970
- TACHIKAWA, HIROTO ET AL: "Hybrid density functional theory (DFT) study on electronic states of halogen-substituted organic-inorganic hybrid compounds: Al-NTCDA" JAPANESE JOURNAL OF APPLIED PHYSICS, PART 1: REGULAR PAPERS, BRIEF COMMUNICATIONS & REVIEW PAPERS , 44(6A), 3769-3773 CODEN: JAPNDE, Juni 2005 (2005-06), XP002429103
- B.A. JONES ET AL: "Cyanonaphthalene Diimide Semiconductors for Air-Stable, Fexible, and Optically Transparent n-Channel Field-Effect Transistors", CHEMISTRY OF MATERIALS, AMERICAN CHEMICAL SOCIETY, WASHINGTON, US, vol. 19, no. 11, 29 May 2007 (2007-05-29), pages 2703-2705, XP002458359, ISSN: 0897-4756, DOI: 10.1021/CM0704579
- C. RÖGER ET AL: "cORE-TETRASUBSTITUTED NAPHTHALENE DIIMIDES", JOURNAL OF ORGANIC CHEMISTRY, vol. 72, 21 September 2007 (2007-09-21), pages 8070-8075,
- S. CHOPIN ET AL.: "Syntheses and properties of core-substituted naphthalene bisimides with aryl ethynyl or cyano groups", JOURNAL OF MATERIALS CHEMISTRY, vol. 17, 23 July 2007 (2007-07-23), pages 4139-4146,
- DATABASE CA [Online] CHEMICAL ABSTRACTS SERVICE, COLUMBUS, OHIO, US 1966:482066 YAKOBSON, G. G. ET AL: 'Preparation of decafluoropyrene and its reaction with nitric acid' Retrieved from STN Database accession no. 1966:482066
- DATABASE CA [Online] CHEMICAL ABSTRACTS SERVICE, COLUMBUS, OHIO, US 1982:544528 MARTYUSHINA, N. V. ET AL: 'Search for nondepolarizing myorelaxants with short duration of action' Retrieved from STN Database accession no. 1982:544528

## Beschreibung

Die vorliegende Erfindung betrifft Naphthalintetracarbonsäurederivate, ein Verfahren zu ihrer Herstellung und deren Verwendung, insbesondere als Halbleiter vom n-Typ.

In vielen Bereichen der Elektronikindustrie werden neben den klassischen anorganischen Halbleitern zunehmend auch organische Halbleiter auf Basis von niedermolekularen oder polymeren Materialien eingesetzt. Diese weisen vielfach Vorteile gegenüber den klassischen anorganischen Halbleitern auf, beispielsweise eine bessere Substratkompatibilität, bessere Verarbeitbarkeit der auf ihnen basierenden Halbleiterbauteile, größere Flexibilität, verringerte Kosten sowie die Möglichkeit, ihre Grenzorbitalenergien mit den Methoden des Molecular Designs auf den jeweiligen Anwendungsbereich genau anzupassen. Ein Hauptanwendungsgebiet in der Elektronikindustrie sind so genannte Feldeffekttransistoren (Field-Effect Transistors, FET). Organischen Feldeffekttransistoren (Organic Field-Effect Transistors, OFET) wird ein großes Entwicklungspotential, beispielsweise in Speicherelementen und integrierten optoelektronischen Vorrichtungen zugeschrieben. Es besteht daher ein großer Bedarf an organischen Verbindungen, die sich als organische Halbleiter, insbesondere Halbleiter vom n-Typ und speziell für einen Einsatz in organischen Feldeffekttransistoren eignen.

Die DE-A-32 35 526 betrifft Perylen-3,4,9,10-tetracarbonsäurediimide, die am Perylengerüst mit Alkoxy- oder Alkylthiogruppen sowie mit Fluor, Chlor oder Brom substituiert sind, ein Verfahren zu ihrer Herstellung und deren Verwendung in lichtsammelnden Kunststoffen.

Die DE-A-195 47 209 betrifft 1,7-Diaryloxy- oder 1,7-Diarylthio-substituierte Perylen-3,4,9,10-tetracarbonsäuren und deren Dianhydride und Diimide. Zu deren Herstellung werden 1,7-Dibromperylen-3,4,9,10-tetracarbonsäuredümide als Zwischenprodukte eingesetzt. Die Herstellung dieser Dibromverbindung gelingt ausgehend vom Perylen-3,4,9,10-tetracarbonsäuredianhydrid durch Bromierung in Gegenwart von lod und Schwefelsäure.

M. J. Ahrens et al. beschreiben in J. Am. Chem. Soc. 2004, 126, S. 8284 - 8294 selbstaufbauende, supermolekulare, lichtsammelnde Arrays aus kovalenten, multi-chromophoren Perylen-3,4,9,10-bis(dicarboximid)-Wiederholungseinheiten.

M. J. Ahrens, M. J. Füller und M. R. Wasielewski beschreiben in Chem. Mater. 2003, 15, S. 2684 - 2686 Cyano-substituierte Perylen-3,4-dicarboximide und Perylen-3,4,9,10-bis(dicarboximide) und deren Einsatz als chromophore Oxidationsmittel, z. B. für einen Einsatz in der Photonik und Elektronik.

B. A. Jones et al. beschreiben in Angew. Chem. 2004, 116, S. 6523 - 6526 den Einsatz von Dicyanoperylen-3,4,9,10-bis(dicarboximiden) als n-Halbleiter.

Die US 2005/0176970 A1 beschreibt n-Halbleiter auf Basis von substituierten Perylen-3,4-dicarboximiden und Perylen-3,4,9,10-bis(dicarboximiden). In diesem Dokument werden ganz allgemein und ohne einen Beleg durch ein Herstellungsbeispiel auch substituierte Naphthalin-1,8-dicarboximide und Naphthalin-1,4,5,8-bis(dicarboximide) und deren Verwendung als n-Halbleiter beschrieben. Als Zwischenstufe zur Herstellung der Perylene werden das 1,7-Dibromperylen-3,4,9,10-tetracarbonsäuredianhydrid und das entsprechende 1,7-Dibromperylen-3,4,9,10-bis(dicarboximid) genannt und bezüglich deren Herstellung auf die Möglichkeit der direkten Bromierung der entsprechenden Kohlenwasserstoffausgangsverbindung Bezug genommen. Anders als die Perylene, sind die entsprechenden 2,6-dibromierten Naphthaline jedoch nicht durch direkte Bromierung, z. B. in Gegenwart von lod und Schwefelsäure, zugängig.

C. H. Thalacker beschreibt in seiner Dissertation, Universität Ulm, 2001, supramolekulare Anordnungen von Napthalin- und Perylenbisimidfarbstoffen auf Basis von Wasserstoff-Brückenbindungen und π-π-Wechselwirkungen. Auf den Seiten 136 und 147 bis 148 dieses Dokuments ist die Synthese von 2,6-Dibromnapthalin-1,8;4,5-tetracarbon-säurebisanhydrid beschrieben, eine Imidierung nur der beiden Anhydridgruppen gelang jedoch nicht.

Die DE 101 48 172 A1 beschreibt Napthalin-1,8;4,5-tetracarbonsäurebisimide der allgemeinen Formel 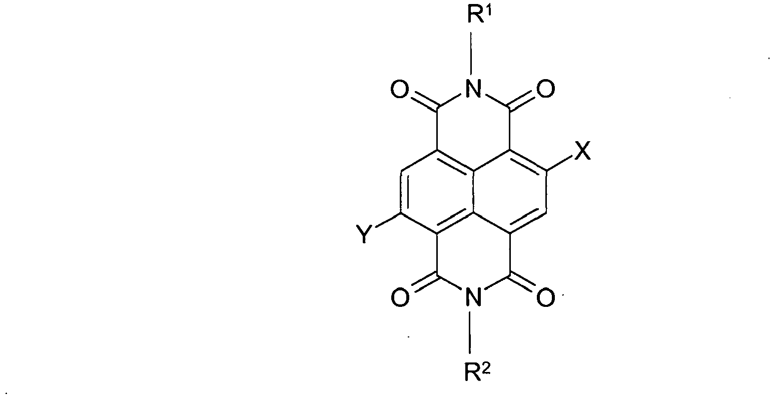 worin
- R¹ und R²: unabhängig voneinander für Wasserstoff, substituiertes oder unsubstituiertes Alkyl oder substituiertes oder unsubstituiertes Aryl stehen und
- X und Y: unabhängig voneinander für Wasserstoff, Halogen, Amino oder einen Rest der Formel -NHR³ oder -OR³ stehen, wobei R³ für -CH₂R⁴, -CHR⁴R⁵ oder -CR⁴R⁵R⁶ steht, wobei R⁴, R⁵, R⁶ unabhängig voneinander für Wasserstoff, substituiertes oder unsubstituiertes Alkyl, Aryl, Alkoxy, Alkylthio, Aryloxy oder Arylthio stehen, wobei mindestens einer der beiden Substituenten X und Y ungleich Wasserstoff, Halogen ist.

Als ein möglicher Ausgangsstoff zur Herstellung dieser Verbindungen wird das 2,6-Dibromnaphthalin-1,8;4,5-tetracarbonsäurebisanhydrid erwähnt und ohne konkrete Angaben auf bekannte Methoden zur Herstellung der entsprechenden 2,5-Dibromnaphthalin-1,8;4,5-tetracarbonsäurediimide verwiesen. Im Widerspruch dazu und in Übereinstimmung mit der o. g. Dissertation von Thalacker beschreibt das Ausführungsbeispiel 4 jedoch die Imidierung von 2,6-Dibromnaphthalin-1,8;4,5-tetra-carbonsäuredianhydrid mit 2-Ethylhexylamin, wobei gleichzeitig die an das aromatische Grundgerüst gebundenen Bromatome durch 2-Ethylhexylaminogruppen substituiert werden. Eine ausführbare Synthese zur Herstellung von 2,6-Dibromnaphthalin-1,8;4,5-tetracarbonsäurediimiden ist somit weder in der DE 101 48 172 A1 noch in der Dissertation von Thalacker offenbart.

H. Tachikawa et al. beschreiben in Japanese Journal of Applied Physics, Bd. 44, Nr. 6A, 2005, S. 3769-3773 Hybriddichtefunktionalrechnungen an Aluminiumkomplexen von Naphthalintetracarbonsäuredianhydrid und halogensubstituierten Naphthalintetracarbonsäuredianhydriden.

DE 37 03 131 A1 beschreibt 2,3,6,7-Tetrafluornaphthalintetracarbonsäurediimide, in denen die Stickstoffatome durch Wasserstoff, Alkyl- oder Arylreste substituiert sind.

Der vorliegenden Erfindung liegt die Aufgabe zu Grunde, neue Verbindungen zur Verfügung zu stellen, die sich als n-Halbleiter, z. B. für einen Einsatz in organischen Feldeffekttransistoren, eignen.

Gelöst wird diese Aufgabe durch eine Verbindung der allgemeinen Formel 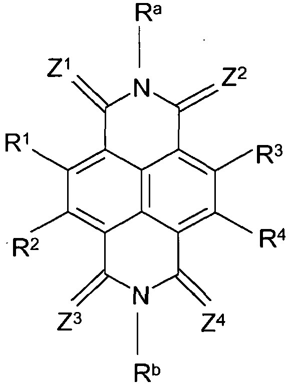 wobei
R¹ und R² für Fluor und R³ und R⁴ für Wasserstoff stehen, oder
R¹ und R³ für Fluor und R² und R⁴ für Wasserstoff stehen, oder
R¹ und R⁴ für Fluor und R² und R³ für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
   oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen,
   worin
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst.

Ein weiterer Gegenstand ist ein Verfahren zur Herstellung von Verbindungen der Formel 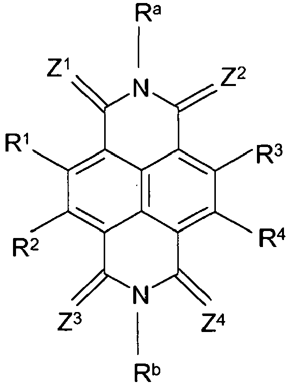 worin
R¹ und R² für Fluor und R³ und R⁴ für Wasserstoff stehen, oder
R¹ und R³ für Fluor und R² und R⁴ für Wasserstoff stehen, oder
R¹ und R⁴ für Fluor und R² und R³ für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
   oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen, bei dem man
   a1) Naphthalin-1,8;4,5-tetracarbonsäuredianhydrid einer Bromierung mit N,N'-Dibromisocyanursäure unter Erhalt einer Verbindung der allgemeinen Formel I.a 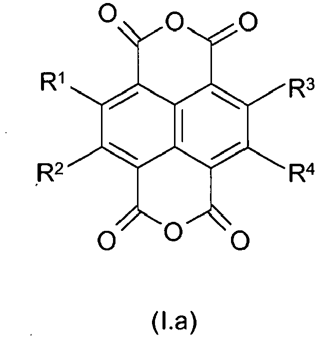 unterzieht, worin zwei oder drei oder vier der Reste R¹, R², R³ und R⁴ für Br stehen, und die übrigen Reste für Wasserstoff stehen,
   b1) die in Schritt a1) erhaltene Verbindung der Formel I.a einer Substitution des Broms durch Fluor sowie gegebenenfalls teilweise durch Wasserstoff, unterzieht,
   c1) die in Schritt b1) erhaltene Verbindung einer Umsetzung mit einem Amin der Formel R^{a}-NH₂ und gegebenenfalls einem Amin der Formel R^{b}-NH₂ unterzieht,
      oder
      die in Schritt b1) erhaltene Verbindung einer Umsetzung mit einem Amin der Formel H₂N-X-NH₂ unterzieht, wobei X für eine zweiwertige verbrückende Gruppe mit 2 bis 5 Atomen zwischen den flankierenden Bindungen steht,
   worin
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst.

Ein weiterer Gegenstand ist ein Verfahren zur Herstellung von Verbindungen der Formel 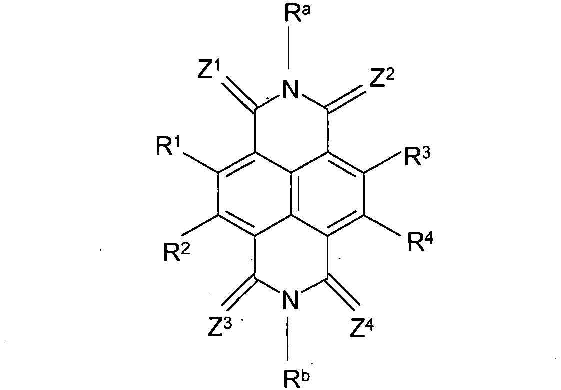 worin
R¹ und R² für Fluor und R³ und R⁴ für Wasserstoff stehen, oder
R¹ und R³ für Fluor und R² und R⁴ für Wasserstoff stehen, oder
R¹ und R⁴ für Fluor und R² und R³ für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
   oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen, bei dem man
   a2) Naphthalin-1,8;4,5-tetracarbonsäuredianhydrid einer Umsetzung mit einem Amin der Formel R^{a}-NH₂ und gegebenenfalls einem Amin der Formel R^{b}-NH₂ unter Erhalt wenigstens einer Verbindung der allgemeinen Formel I.a21) 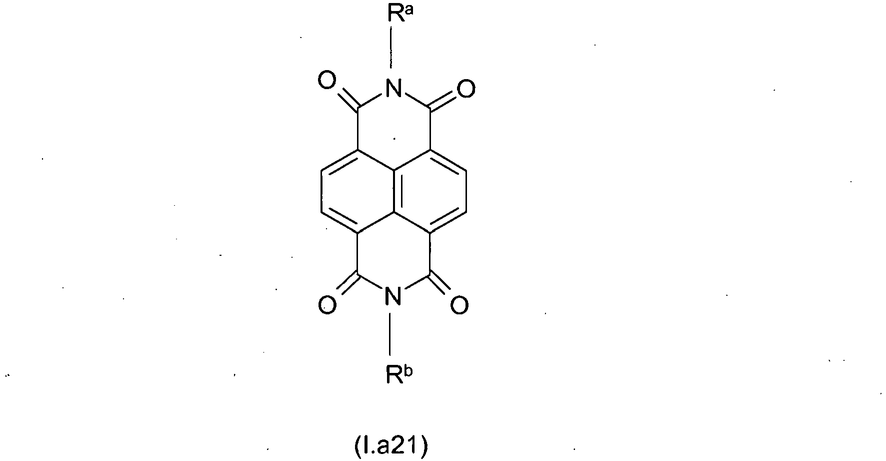 unterzieht, wobei R^{b} auch die gleiche Bedeutung wie R^{a} besitzen kann,
      oder
      Naphthalin-1,8;4,5-tetracarbonsäuredianhydrid einer Umsetzung mit einem Amin der Formel H₂N-X-NH₂ unter Erhalt wenigstens einer Verbindung der allgemeinen Formel I.a22) 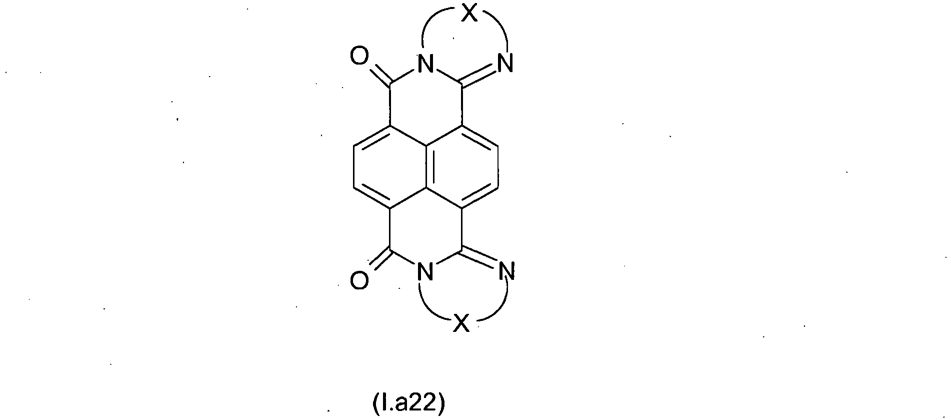 oder einem Isomeren davon unterzieht, wobei X für eine zweiwertige verbrückende Gruppe mit 2 bis 5 Atomen zwischen den flankierenden Bindungen steht.
   b2) die in Schritt a2) erhaltene(n) Verbindung(en) einer Bromierung mit N,N'-Dibromisocyanursäure unterzieht,
   c2) die in Schritt b2) erhaltene(n) Verbindung(en) einer Substitution des Broms durch Fluor sowie gegebenenfalls teilweise durch Wasserstoff, unterzieht,
   worin
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst.

Ein weiterer Gegenstand ist die Verwendung von Verbindungen der Formel 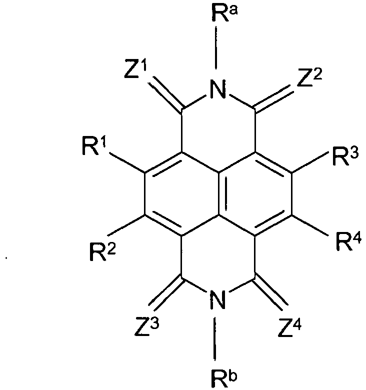 worin
wenigstens einer der Reste R¹, R², R³ und R⁴ für einen Substituenten steht, der ausgewählt ist unter Br, F und CN, und die übrigen Reste für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
   oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen,
als Halbleiter, insbesondere für organische Feldeffekttransistoren und die organische Photovoltaik, worin
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst.

Im Rahmen der vorliegenden Erfindung umfasst der Ausdruck "Alkyl" geradkettiges oder verzweigtes Alkyl. Vorzugsweise handelt es sich um geradkettiges oder verzweigtes C₁-C₃₀-Alkyl, insbesondere um C₁-C₂₀-Alkyl und ganz besonders bevorzugt C₁-C₁₂-Alkyl. Beispiele für Alkylgruppen sind insbesondere Methyl, Ethyl, Propyl, Isopropyl, n-Butyl, Isobutyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl- 2-methylpropyl, n-Heptyl, 2-Heptyl, 3-Heptyl, 2-Ethylpentyl, 1-Propylbutyl, n-Octyl, 2-Ethylhexyl, 2-Propylheptyl, 1,1,3,3-Tetramethylbutyl, Nonyl, Decyl, n-Undecyl, n-Dodecyl, n-Tridecyl, iso-Tridecyl, n-Tetradecyl, n-Hexadecyl, n-Octadecyl und n,-Eicosyl.

Der Ausdruck Alkyl umfasst auch Alkylreste, deren Kohlenstoffketten durch eine oder mehrere nicht benachbarte Gruppen, die ausgewählt sind unter -O-, -S-, -NR^{e}-, -CO- und/oder -SO₂- unterbrochen sein kann, d.h. die Termini der Alkylgruppe werden durch Kohlenstoffatome gebildet. R^{e} steht vorzugsweise für Wasserstoff, Alkyl, Cycloalkyl, Heterocycloalkyl, Aryl oder Hetaryl.

Die vorstehenden Ausführungen zu Alkyl gelten auch für die Alkylteile in Alkoxy, Alkylamino, Alkylthio, Alkylsulfinyl und Alkylsulfonyl.

Der Ausdruck "Alkenyl" umfasst im Sinne der vorliegenden Erfindung geradkettige und verzweigte Alkenylgruppen, die in Abhängigkeit von der Kettenlänge eine oder mehrere Doppelbindungen tragen können. Bevorzugt sind C₂-C₂₀-, besonders bevorzugt C₂-C₁₀-Alkenylgruppen. Der Ausdruck "Alkenyl" umfasst auch substituierte Alkenylgruppen, welche z. B. 1, 2, 3, 4 oder 5 Substituenten tragen können. Geeignete Substituenten sind z. B. Cycloalkyl, Heterocycloalkyl, Aryl, Heteroaryl, Nitro, Cyano, Halogen, Amino, Mono- oder Di-(C₁-C₂₀-Alkyl)amino.

Alkenyl steht dann beispielsweise für Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1 ,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1,3-Butadienyl, 1-Methyl-1,3-butadienyl, 2-Methyl-1,3-butadienyl, Penta-1,3-dien-1-yl, Hexa-1,4-dien-1-yl, Hexa-1,4-dien-3-yl, Hexa-1,4-dien-6-yl, Hexa-1,5-dien-1-yl, Hexa-1,5-dien-3-yl, Hexa-1,5-dien-4-yl, Hepta-1,4-dien-1-yl, Hepta-1,4-dien-3-yl, Hepta-1,4-dien-6-yl, Hepta-1,4-dien-7-yl, Hepta-1,5-dien-1-yl, Hepta-1,5-dien-3-yl, Hepta-1,5-dien-4-yl, Hepta-1,5-dien-7-yl, Hepta-1,6-dien-1-yl, Hepta-1,6-dien-3-yl, Hepta-1,6-dien-4-yl, Hepta-1,6-dien-5-yl, Hepta-1,6-dien-2-yl, Octa-1,4-dien-1-yl, Octa-1,4-dien-2-yl, Octa-1,4-dien-3-yl, Octa-1,4-dien-6-yl, Octa-1,4-dien-7-yl, Octa-1,5-dien-1-yl, Octa-1,5-dien-3-yl, Octa-1,5-dien-4-yl, Octa-1,5-dien-7-yl, Octa-1,6-dien-1-yl, Octa-1,6-dien-3-yl, Octa-1,6-dien-4-yl, Octa-1,6-dien-5-yl, Octa-1,6-dien-2-yl, Deca-1,4-dienyl, Deca-1,5-dienyl, Deca-1,6-dienyl, Deca-1,7-dienyl, Deca-1,8-dienyl, Deca-2,5-dienyl, Deca-2,6-dienyl, Deca-2,7-dienyl, Deca-2,8-dienyl und dergleichen. Die Ausführungen zu Alkenyl gelten auch für die Alkenylgruppen in Alkenyloxy, Alkenylthio, etc.

Der Ausdruck "Alkinyl" umfasst unsubstituierte oder substituierte Alkinylgruppen, die eine oder mehrere nicht benachbarte Dreifachbindungen aufweisen, wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl und dergleichen. Die Ausführungen zu Alkinyl gelten auch für die Alkinylgruppen in Alkinyloxy, Alkinylthio, etc.

Der Ausdruck "Cycloalkyl" umfasst im Rahmen der vorliegenden Erfindung sowohl unsubstituierte als auch substituierte Cycloalkylgruppen, vorzugsweise C₃-C₈-Cycloalkylgruppen wie Cyclopropyl, Cyclobutyl, Cyclopentyl, Cyclohexyl, Cycloheptyl oder Cyclooctyl, insbesondere C₅-C₈-Cycloalkyl. Die Cycloalkylgruppen können im Falle einer Substitution einen oder mehrere, beispielsweise eine, zwei, drei, vier oder fünf C₁-C₆-Alkylgruppen tragen.

C₅-C₈-Cycloalkyl, das unsubstituiert ist oder eine oder mehrere C₁-C₆-Alkylgruppen tragen kann, steht beispielsweise für Cyclopentyl, 2- und 3-Methylcyclopentyl, 2- und 3-Ethylcyclopentyl, Cyclohexyl, 2-, 3- und 4-Methylcyclohexyl, 2-, 3- und 4-Ethylcyclohexyl, 3- und 4-Propylcyclohexyl, 3- und 4-Isopropylcyclohexyl, 3- und 4-Butylcyclohexyl, 3- und 4-sec.-Butylcyclohexyl, 3- und 4-tert.-Butylcyclohexyl, Cycloheptyl, 2-, 3- und 4-Methylcycloheptyl, 2-, 3- und 4-Ethylcycloheptyl, 3- und 4-Propylcycloheptyl, 3- und 4-Isopropylcycloheptyl, 3- und 4-Butylcycloheptyl, 3- und 4-sec.-Butylcycloheptyl, 3- und 4-tert.-Butylcycloheptyl, Cyclooctyl, 2-, 3-, 4- und 5-Methylcyclooctyl, 2-, 3-, 4- und 5-Ethylcyclooctyl, 3-, 4- und 5-Propylcyclooctyl.

Der Ausdruck Cycloalkenyl umfasst vorzugsweise monocyclische, einfach ungesättigte Kohlenwasserstoffgruppen mit 3 bis 8, vorzugsweise 5 bis 6 Kohlenstoffringgliedern, wie Cyclopenten-1-yl, Cyclopenten-3-yl, Cyclohexen-1-yl, Cyclohexen-3-yl, Cyclohexen-4-yl und dergleichen.

Der Ausdruck Bicycloalkyl umfasst vorzugsweise bicyclische Kohlenwasserstoffreste mit 5 bis 10 C-Atomen wie Bicyclo[2.2.1]hept-1-yl, Bicyclo[2.2.1]hept-2-yl, Bicyclo[2.2.1]hept-7-yl, Bicyclo[2.2.2]oct-1-yl, Bicyclo[2.2.2]oct-2-yl, Bicyclo[3.3.0]octyl, Bicyclo[4.4.0]decyl und dergleichen.

Der Ausdruck "Aryl" umfasst im Rahmen der vorliegenden Erfindung ein- oder mehrkernige aromatische Kohlenwasserstoffreste, die unsubstituiert oder substituiert sein können. Der Ausdruck "Aryl" steht vorzugsweise für Phenyl, Tolyl, Xylyl, Mesityl, Duryl, Naphthyl, Fluorenyl, Anthracenyl, Phenanthrenyl oder Naphthyl, besonders bevorzugt für Phenyl oder Naphthyl, wobei diese Arylgruppen im Falle einer Substitution im Allgemeinen 1, 2, 3, 4 oder 5, vorzugsweise 1, 2 oder 3 Substituenten tragen können, die ausgewählt sind unter C₁-C₁₈-Alkyl, C₁-C₆-Alkoxy, Cyano, CONR^{f}R^{g}, CO₂R^{f}, Arylazo und Heteroarylazo, wobei Arylazo und Heteroarylazo ihrerseits unsubstituiert sind oder einen oder mehrere Reste tragen, die unabhängig voneinander ausgewählt sind unter C₁-C₁₈-Alkyl, C₁-C₆-Alkoxy und Cyano. R^{f} und R^{g} stehen vorzugsweise unabhängig voneinander für Wasserstoff, Alkyl, Cycloalkyl, Heterocycloalkyl, Aryl oder Hetaryl.

Aryl, das unsubstituiert ist oder einen oder mehrere Reste trägt, die unabhängig voneinander ausgewählt sind unter C₁-C₁₈-Alkyl, C₁-C₆-Alkoxy und Cyano steht beispielsweise für 2-, 3- und 4-Methylphenyl, 2,4-, 2,5-, 3,5- und 2,6-Dimethylphenyl, 2,4,6-Trimethylphenyl, 2-, 3- und 4-Ethylphenyl, 2,4-, 2,5-, 3,5- und 2,6-Diethylphenyl, 2,4,6-Triethylphenyl, 2-, 3- und 4-Propylphenyl, 2,4-, 2,5-, 3,5- und 2,6- Dipropylphenyl, 2,4,6-Tripropylphenyl, 2-, 3- und 4-Isopropylphenyl, 2,4-, 2,5-, 3,5- und 2,6-Diisopropylphenyl, 2,4,6-Triisopropylphenyl, 2-, 3- und 4-Butylphenyl, 2,4-, 2,5-, 3,5- und 2,6-Dibutylphenyl, 2,4,6-Tributylphenyl, 2-, 3- und 4-Isobutylphenyl, 2,4-, 2,5-, 3,5- und 2,6-Diisobutylphenyl, 2,4,6-Triisobutylphenyl, 2-, 3- und 4-sec-Butylphenyl, 2,4-, 2,5-, 3,5- und 2,6-Di-sec-butylphenyl, 2,4,6-Tri-sec-butylphenyl, 2-, 3- und 4-tert.-Butylphenyl, 2,4-, 2,5-, 3,5- und 2,6-Di-tert.-butylphenyl und 2,4,6-Tri-tert.-butylphenyl; 2-, 3- und 4-Methoxyphenyl, 2,4-, 2,5-, 3,5- und 2,6-Dimethoxyphenyl, 2,4,6-Trimethoxyphenyl, 2-, 3- und 4-Ethoxyphenyl, 2,4-, 2,5-, 3,5- und 2,6-Diethoxyphenyl, 2,4,6-Triethoxyphenyl, 2-, 3- und 4-Propoxyphenyl, 2,4-, 2,5-, 3,5- und 2,6-Dipropoxyphenyl, 2-, 3- und 4-Isopropoxyphenyl, 2,4-, 2,5-, 3,5- und 2,6-Diisopropoxyphenyl und 2-, 3- und 4-Butoxyphenyl; 2-, 3- und 4-Cyanophenyl.

Der Ausdruck "Heterocycloalkyl" umfasst im Rahmen der vorliegenden Erfindung nicht-aromatische, ungesättigte oder vollständig gesättigte, cycloaliphatische Gruppen mit im Allgemeinen 5 bis 8 Ringatomen, vorzugsweise 5 oder 6 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind und das unsubstituiert ist oder mit einer oder mehreren, beispielsweise 1,2,3,4,5 oder 6 C₁-C₆-Alkylgruppen substituiert ist. Beispielhaft für solche heterocycloaliphatischen Gruppen seien Pyrrolidinyl, Piperidinyl, 2,2,6,6-Tetramethyl-piperidinyl, Imidazolidinyl, Pyrazolidinyl, Oxazolidinyl, Morpholidinyl, Thiazolidinyl, Isothiazolidinyl, Isoxazolidinyl, Piperazinyl-, Tetrahydrothiophenyl, Dihydrothien-2-yl, Tetrahydrofuranyl, Dihydrofuran-2-yl, Tetrahydropyranyl, 1,2-Oxazolin-5-yl, 1,3-Oxazolin-2-yl und Dioxanyl genannt.

Der Ausdruck "Heteroaryl" umfasst im Rahmen der vorliegenden Erfindung unsubstituierte oder substituierte, heteroaromatische, ein- oder mehrkernige Gruppen, vorzugsweise die Gruppen Pyridyl, Chinolinyl, Acridinyl, Pyridazinyl, Pyrimidinyl, Pyrazinyl, Pyrrolyl, Imidazolyl, Pyrazolyl, Indolyl, Purinyl, Indazolyl, Benzotriazolyl, 1,2,3-Triazolyl, 1,3,4-Triazolyl und Carbazolyl, wobei diese heterocycloaromatischen Gruppen im Falle einer Substitution im Allgemeinen 1, 2 oder 3 Substituenten tragen können. Die Substituenten sind ausgewählt unter C₁-C₆-Alkyl, C₁-C₆-Alkoxy, Hydroxy, Carboxy und Cyano.

Über ein Stickstoffatom gebundene 5- bis 7-gliedrige Heterocycloalkyl- oder Heteroarylreste, die gegebenenfalls weitere Heteroatome enthalten, stehen beispielsweise für Pyrrolyl, Pyrazolyl, Imidazolyl, Triazolyl, Pyrrolidinyl, Pyrazolinyl, Pyrazolidinyl, Imidazolinyl, Imidazolidinyl, Pyridinyl, Pyridazinyl, Pyrimidinyl, Pyrazinyl, Triazinyl, Piperidinyl, Piperazinyl, Oxazolyl, Isooxazolyl, Thiazolyl, Isothiazolyl, Indolyl, Chinolinyl, Isochinolinyl oder Chinaldinyl.

Halogen steht für Fluor, Chlor, Brom oder lod.

Konkrete Beispiele für die in den folgenden Formeln genannten Reste sowie deren Substituenten sind im Einzelnen:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den nach der Oxosynthese erhaltenen Alkoholen);
Ethinyl, 1- und 2-Propinyl, 1-, 2- und 3-Butinyl, 1-, 2-, 3- und 4-Pentinyl, 1-, 2-, 3-, 4-und 5-Hexinyl, 1-, 2-, 3-, 4-, 5-, 6-, 7-, 8- und 9-Decinyl, 1-, 2-, 3-, 4-, 5-, 6-, 7-, 8-, 9-, 10- und 11-Dodecinyl und 1-, 2-, 3-, 4-, 5-, 6-, 7-, 8-, 9-, 10-, 11-, 12-, 13-, 14-, 15-, 16-und 17-Octadecinyl;
Ethenyl, 1- und 2-Propenyl, 1-, 2- und 3-Butenyl, 1-, 2-, 3- und 4-Pentenyl, 1-, 2-, 3-, 4-und 5-Hexenyl, 1-, 2-, 3-, 4-, 5-, 6-, 7-, 8- und 9-Decenyl, 1-, 2-, 3-, 4-, 5-, 6-, 7-, 8-, 9-, 10- und 11-Dodecenyl und 1-, 2-, 3-, 4-, 5-, 6-, 7-, 8-, 9-, 10-, 11-, 12-, 13-, 14-, 15-, 16-und 17-Octadecenyl; Cyclopropyl, Cyclobutyl, Cyclopentyl, 2Cyclohexyl, Cycloheptyl, Cyclooctyl;
1-, 2- und 3-Cyclopentenyl, 1-, 2-, 3- und 4-Cyclohexenyl, 1-, 2- und 3-Cycloheptenyl und 1-, 2-, 3- und 4-Cyclooctenyl;
2-Dioxanyl, 1-Morpholinyl, 1-Thiomorpholinyl, 2- und 3-Tetrahydrofuryl, 1-, 2- und 3-Pyrrolidinyl, 1-Piperazyl, 1-Diketopiperazyl und 1-, 2-, 3- und 4-Piperidyl;
Phenyl, 2-Naphthyl, 2- und 3-Pyrryl, 2-, 3- und 4-Pyridyl, 2-, 4- und 5-Pyrimidyl, 3-, 4-und 5-Pyrazolyl, 2-, 4- und 5-lmidazolyl, 2-, 4- und 5-Thiazolyl, 3-(1,2,4-Triazyl), 2-(1,3,5-Triazyl), 6-Chinaldyl, 3-, 5-, 6- und 8-Chinolinyl, 2-Benzoxazolyl, 2-Benzothiazolyl, 5-Benzothiadiazolyl, 2- und 5-Benzimidazolyl und 1- und 5-Isochinolyl;
1-, 2-, 3-, 4-, 5-, 6- und 7-Indolyl, 1-, 2-, 3-, 4-, 5-, 6- und 7-Isoindolyl, 1-, 2-, 4-, 6-, 7-und 8-(1,2,3,4-Tetrahydroisochinolinyl), 31-, 2-, 3-, 4-, 5-, 6-, 7- und 8-(1,2,3,4-Tetrahydrochinolinyl) und 2-, 3-, 4-, 5-, 6-, 7- und 8-Chromanyl, 2-, 4- und 7-Chinolinyl.

Bevorzugt verwendet werden Verbindungen der Formel , wobei R¹, R², R³ und R⁴ alle für Brom oder alle für Fluor oder alle für Cyano stehen.

Des Weiteren bevorzugt sind Verbindungen, wobei R¹ und R² beide für Fluor und R³ und R⁴ für Wasserstoff stehen.

Des Weiteren bevorzugt sind Verbindungen, wobei R¹ und R³ beide für Fluor und R² und R⁴ für Wasserstoff stehen.

Des Weiteren bevorzugt sind Verbindungen, wobei R¹ und R⁴ beide für Fluor und R² und R³ für Wasserstoff stehen.

In einer weiteren speziellen Ausführungsform sind die Reste R^{a} und R^{b} gleich.

Besonders bevorzugt sind weiterhin Verbindungen der allgemeinen Formel I.b 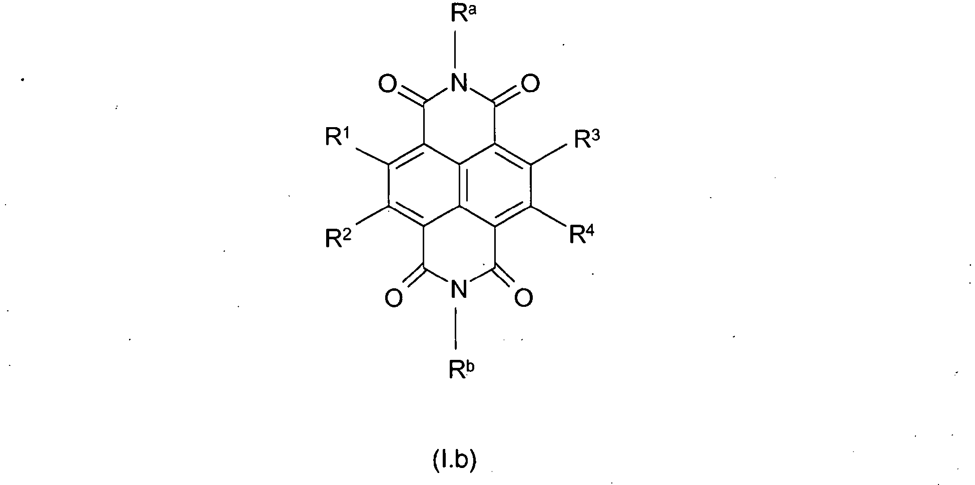 worin
R¹, R², R³ und R⁴ die zuvor angegebenen Bedeutungen besitzen und
R^{a} und R^{b} unabhängig voneinander eine der zuvor genannten Bedeutungen aufweisen.

Bezüglich geeigneter und bevorzugter Reste R¹, R², R³, R⁴, R^{a} und R^{b} wird auf die vorherigen Ausführungen in vollem Umfang Bezug genommen.

Eine weitere bevorzugte Ausführungsform sind Verbindungen der allgemeinen Formel I.c 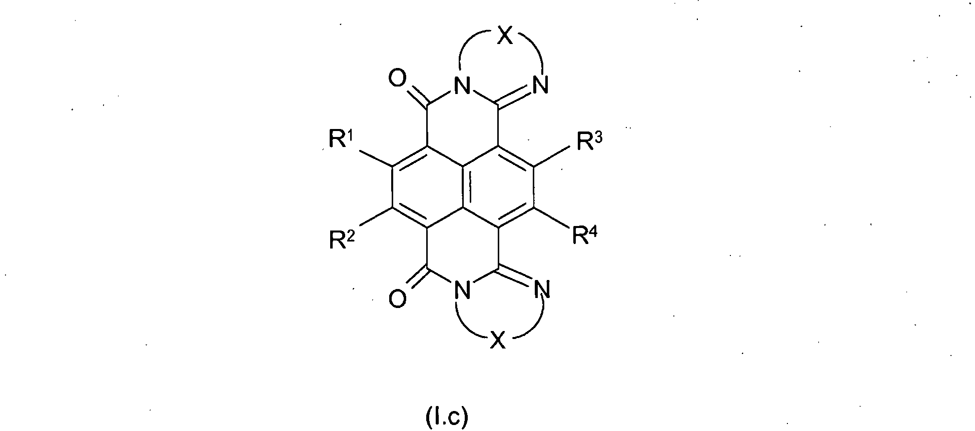 wobei
R¹, R², R³ und R⁴ die zuvor angegebenen Bedeutungen besitzen und
X für eine zweiwertige verbrückende Gruppe mit 2 bis 5 Atomen zwischen den flankierenden Bindungen steht,
und die Strukturisomeren davon.

Bevorzugt stehen die verbrückenden Gruppen X gemeinsam mit der N-C=N-Gruppe, an die sie gebunden sind, für einen 5- bis 8-gliedrigen Heterocyclus, der gegebenenfalls ein-, zwei- oder dreifach mit Cycloalkyl, Heterocycloalkyl, Aryl und/oder Hetaryl anelliert ist, wobei die anellierten Gruppen unabhängig voneinander je einen, zwei, drei oder vier Substituenten, ausgewählt unter Alkyl, Alkoxy, Cycloalkyl, Aryl, Halogen, Hydroxy, Thiol, Polyalkylenoxid, Polyalkylenimin, COOH, Carboxylat, SO₃H, Sulfonat, NE¹E², Alkylen-NE¹E², Nitro und Cyano tragen können, wobei E¹ und E² unabhängig voneinander für Wasserstoff, Alkyl, Cycloalkyl, Heterocycloalkyl, Aryl oder Hetaryl stehen, und/oder X einen, zwei oder drei Substituenten, die ausgewählt sind unter gegebenenfalls substituiertem Alkyl, gegebenenfalls substituiertem Cycloalkyl und gegebenenfalls substituiertem Aryl, aufweisen kann und/oder X durch 1, 2 oder 3 gegebenenfalls substituierte Heteroatome unterbrochen sein kann.

Vorzugsweise sind die verbrückenden Gruppen X ausgewählt unter Gruppen der Formeln 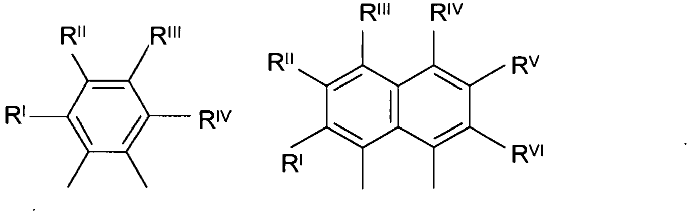 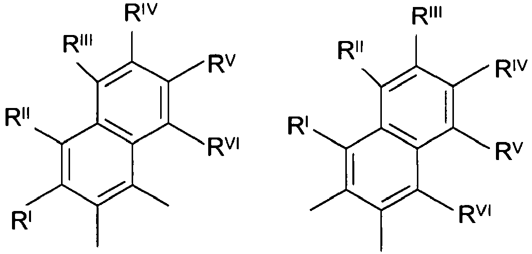 worin
R¹, R", R^{III}, R^{IV}, R^{V} und R^{VI} unabhängig voneinander für Wasserstoff, Alkyl, Alkoxy, Cycloalkyl, Cycloalkoxy, Heterocycloalkyl, Heterocycloalkoxy, Aryl, Aryloxy, Hetaryl, Hetaryloxy, Halogen, Hydroxy, Thiol, Polyalkylenoxid, Polyalkylenimin, COOH, Carboxylat, SO₃H, Sulfonat, NE¹E², Alkylen-NE¹E², Nitro, Alkoxycarbonyl, Acyl oder Cyano stehen, wobei E¹ und E² unabhängig voneinander für Wasserstoff, Alkyl, Cycloalkyl, Heterocycloalkyl, Aryl oder Hetaryl stehen.

Im Folgenden werden einige besonders bevorzugt erfindungsgemäß verwendete Verbindungen wiedergegeben:

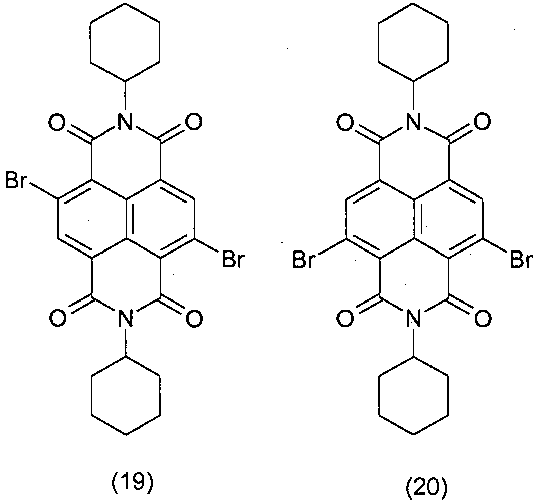

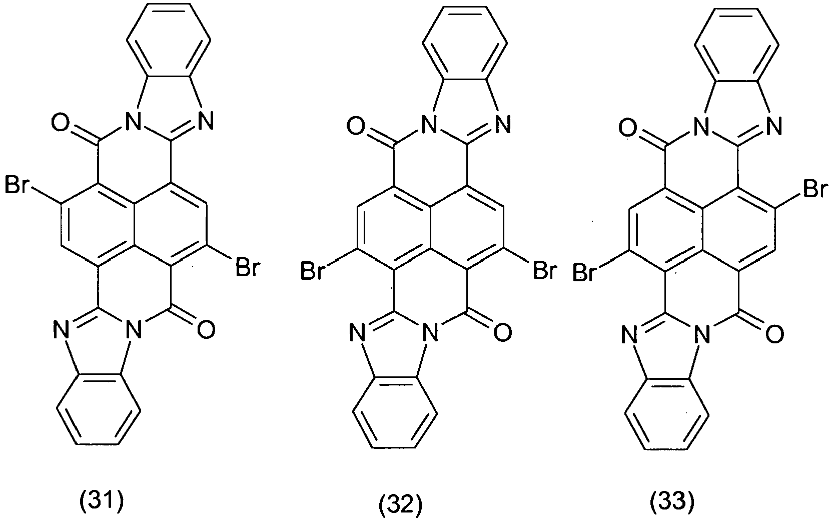

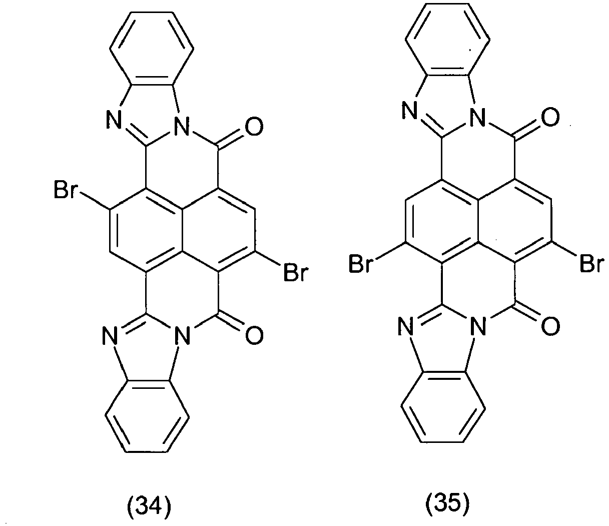

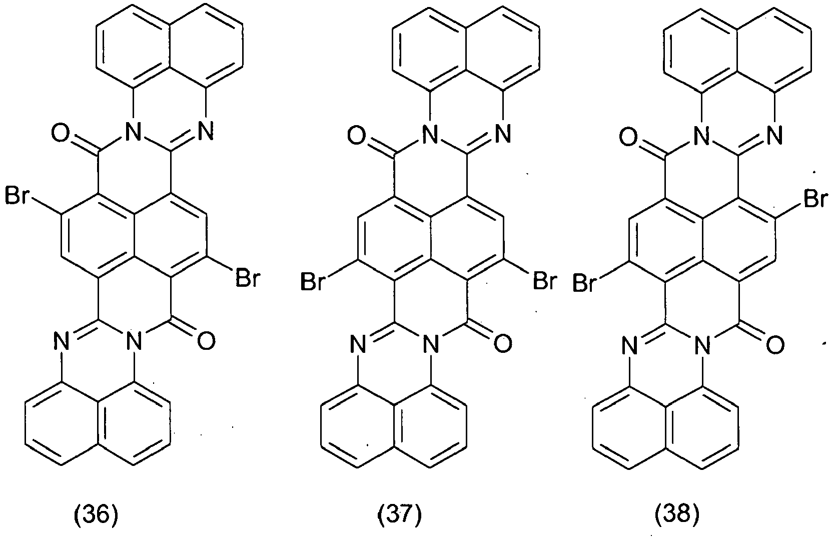

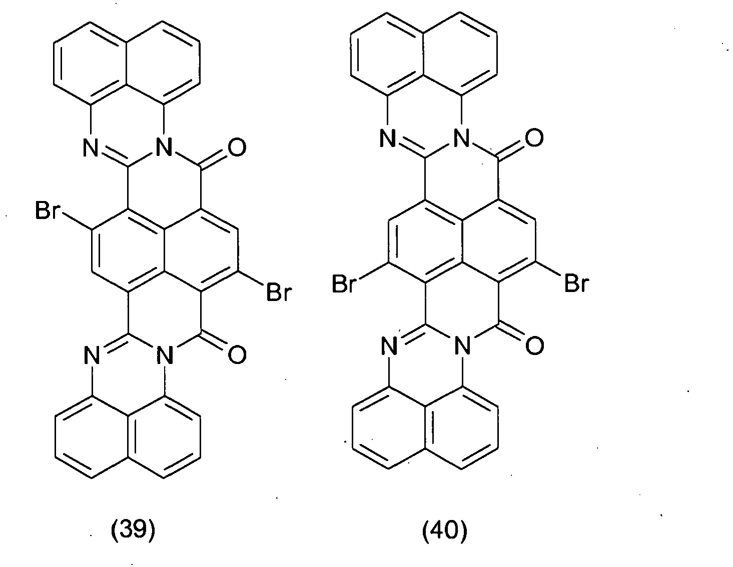

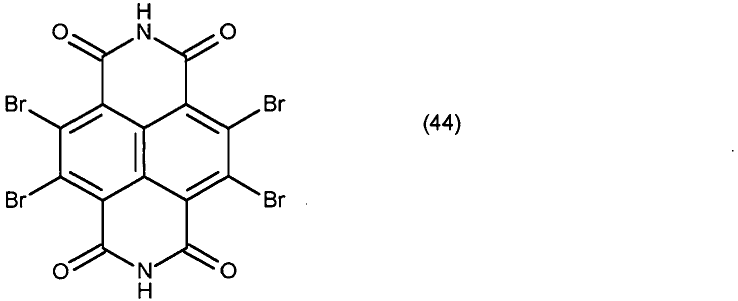

Es wurde jetzt überraschenderweise gefunden, dass man durch die Verwendung von Oleum, welches eine Konzentration von mehr als 20 %, und insbesondere von wenigstens 28 %, aufweist (z. B. durch Verwendung von 30%igem Oleum), als Lösungsmittel bei der Bromierung von Naphthalintetracarbonsäurebisanhydrid überraschenderweise das Tetrabrombisanhydrid erhält. Darüber hinaus wurde gefunden, dass man dieses Tetrabrombisanhydrid
- einer vollständigen Substitution der Bromatome durch Fluoratome oder Cyanogruppen,
- einer teilweisen Substitution der Bromatome durch Wasserstoff, oder
- einer teilweisen Substitution der Bromatome durch Fluoratome oder Cyanogruppen bei gleichzeitiger teilweiser Substitution durch Wasserstoff
unterziehen kann.

Beschrieben wird daher ein Verfahren zur Herstellung von Verbindungen der Formel I.a 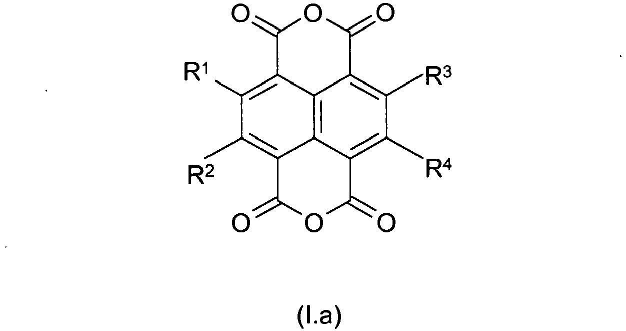 worin
wenigstens einer der Reste R¹, R², R³ und R⁴ für einen Substituenten steht, der ausgewählt ist unter Br, F und CN, und die übrigen Reste für Wasserstoff stehen,
bei dem man
   i) Naphthalin-1,8;4,5-tetracarbonsäuredianhydrid einer Bromierung mit Dibromisocyanursäure in Gegenwart von mehr als 20%igem Oleum unter Erhalt des 2,3,6,7-Tetrabromnaphthalin-1,8;4,5-tetracarbonsäuredianhydrids 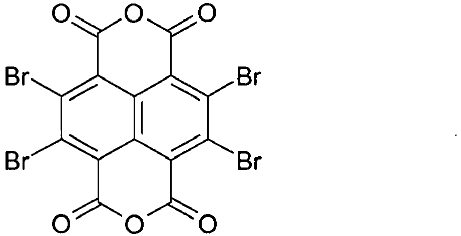 unterzieht,
   ii) gegebenenfalls das 2,3,6,7-Tetrabromnaphthalin-1,8;4,5-tetracarbonsäuredianhydrid einer Substitution der Bromatome durch Fluor oder durch Cyanogruppen oder das 2,3,6,7-Tetrabromnaphthalin-1,8;4,5-tetracarbonsäuredianhydrid einer teilweisen Substitution der Bromatome durch Wasserstoff und gegebenenfalls durch Fluor oder durch Cyanogruppen unterzieht,
   iii) gegebenenfalls die in Schritt ii) erhaltenen Verbindungen wenigstens einem Auftrennungs- und/oder Reinigungsschritt unterzieht.

### Schritt i)

Zur Umsetzung in Schritt i) wird vorzugsweise wenigstens 25%iges, insbesondere wenigstens 28%iges Oleum (wie z. B. 30%iges Oleum) eingesetzt.

Das Molmengenverhältnis von Dibromisocyanursäure zu Naphthalin-1,8;4,5-tetracarbonsäurebisanhydrid liegt vorzugsweise in einem Bereich von etwa 4 : 1 bis 0,9 : 1, besonders bevorzugt 3 : 1 bis 0,9 : 1. Das Molmengenverhältnis beträgt insbesondere 4 : 1 bis 1,5 : 1, speziell 2,5 : 1 bis 1,5 : 1.

### Schritt ii)

Geeignete Verfahrensbedingungen zur aromatischen nucleophilen Substitution von Bromatomen oder Chloratomen durch Fluoratome (Halo-Dehalogenierung) sind prinzipiell bekannt. Geeignete Bedingungen zur Halo-Dehalogenierung sind z. B. in J. March, Advanced Organic Chemistry, 4. Auflage, Verlag John Wiley & Sons (1992), S. 659 sowie in der DE-A-32 35 526 beschrieben. Auf diese Offenbarung wird hier Bezug genommen.

In einer ersten Ausführungsform handelt es sich bei der Umsetzung in Schritt ii) um einen Austausch der Bromatome durch Fluoratome, gegebenenfalls mit einer teilweisen Dehalogenierung. Zur Einfügung der Fluorgruppen wird vorzugsweise ein Alkalifluorid, insbesondere KF, NaF oder CsF, eingesetzt.

Bevorzugte Lösungsmittel für den Halogenaustausch in Schritt ii) sind aprotisch polare Lösungsmittel, wie Dimethylformamid, N-Methylpyrrolidon, (CH₃)₂SO, Dimethylsulfon oder insbesondere Sulfolan. Vorzugsweise werden die Lösungsmittel vor ihrem Einsatz einer Trocknung zur Entfernung von Wasser nach üblichen, dem Fachmann bekannten Methoden unterzogen. Das gilt speziell für die Entfernung von Restmengen an Wasser aus Sulfolan.

Zum Halogenaustausch in Schritt ii) kann zusätzlich ein Komplexbildner, wie z. B. ein Kronenether eingesetzt werden. Dazu zählen z. B. [12]Krone-4, [15]Krone-5, [18]Krone-6, [21]Krone-7, [24]Krone-8, etc. Die Auswahl des Komplexbildners erfolgt nach seiner Befähigung zur Komplexierung der Alkalimetalle der zum Halogenaustausch eingesetzten Alkalihalogenide. Wird zur Einfügung der Fluorgruppen KF eingesetzt, so wird als Komplexbildner bevorzugt [18]Krone-6 eingesetzt.

Weitere geeignete Phasentransferkatalysatoren sind zum Einsatz in Schritt ii) beispielsweise ausgewählt unter 2-Azaalleniumverbindungen, Carbophosphazeniumverbindungen, Aminophosphoniumverbindungen und Diphosphazeniumverbindungen. A. Pleschke, A. Marhold, M. Schneider, A. Kolomeitsev und G. V. Röschenthaler geben im Journal of Fluorine Chemistry 125, 2004, 1031-1038 eine Übersicht über weitere geeignete Phasentransferkatalysatoren. Auf die Offenbarung dieses Dokuments wird Bezug genommen. In einer bevorzugten Ausführung werden 2-Azaalleniumverbindungen, wie (N,N-Dimethylimidazolidino)tetramethylguanidiniumchlorid (CNC⁺), eingesetzt. Besonders bevorzugt wird dann Sulfolan als Lösungsmittel eingesetzt. Die Einsatzmenge der zuvor genannten Phasentransferkatalysatoren beträgt vorzugsweise 0,1 bis 20 Gew.-%, bevorzugt 1 bis 10 Gew.-%, bezogen auf das Gewicht der eingesetzten Rylenverbindung.

Bei der Umsetzung mit Alkalifluoriden in einem wasserfreien, aprotischen, polaren Lösungsmittel erfolgt neben einem Halogenaustausch im Allgemeinen auch zu einem gewissen Grad eine Dehalogenierung. Die dabei erhaltenen Gemische aus Dihalogen-, Trihalogen- und/oder Tetrahalogennapthalintetracarbonsäureanhydriden können im Folgenden einer Auftrennung unterzogen werden.

In einer weiteren Ausführungsform handelt es sich bei der Umsetzung in Schritt ii) um einen Austausch der Bromatome durch Cyanogruppen, gegebenenfalls mit einer teilweisen Dehalogenierung. Geeignete Verfahrensbedingungen zur Cyano-Dehalogenierung sind ebenfalls in J. March, Advanced Organic Chemistry, 4. Auflage, Verlag John Wiley & Sons (1992), S. 660 - 661 sowie in der WO 2004/029028 beschrieben. Dazu zählt beispielsweise die Umsetzung mit Kupfercyanid. Geeignet sind weiterhin Alkalicyanide, wie KCN und NaCN, sowie Zinkcyanid in polaren aprotischen Lösungsmitteln in Gegenwart von Pd(II)salzen oder Kupfer oder Nickelkomplexen. Bevorzugte polare aprotische Lösungsmittel sind die zuvor für den Halogenaustausch Genannten.

Die Auftrennung und/oder Reinigung in Schritt iii) kann nach üblichen, dem Fachmann bekannten Verfahren erfolgen, wie Extraktion, Destillation, Umkristallisation, Auftrennung an geeigneten stationären Phasen sowie einer Kombination dieser Maßnahmen.

Überraschenderweise wurde gefunden, dass bisher nicht zugängige halogensubstituierte Naphthalin-1,8;4,5-tetracarbonsäurediimide erhalten werden können, wenn man Naphthalin-1,8:4,5-tetracarbonsäuredianhydrid zunächst einer Bromierung, dann einer Substitution des Broms durch Fluor (gegebenenfalls verbunden mit einer teilweisen Dehalogenierung) und anschließend einer Imidierung unterzieht. Weiterhin wurde gefunden, dass bisher nicht zugängige halogensubstituierte Naphthalin-1,8;4,5-tetracarbonsäurediimide erhalten werden können, wenn man Naphthalin-1,8;4,5-tetracarbonsäuredianhydrid zunächst einer Imidierung, dann einer Bromierung und anschließend einer Substitution des Broms durch Fluor (gegebenenfalls verbunden mit einer teilweisen Dehalogenierung) unterzieht. In beiden Verfahrensvarianten wird eine Imidierung eines bromsubstituierten Naphthalin-1,8;4,5-tetracarbonsäuredianhydrids vermieden.

Ein weiterer Gegenstand der Erfindung ist daher ein Verfahren zur Herstellung von Verbindungen der Formel 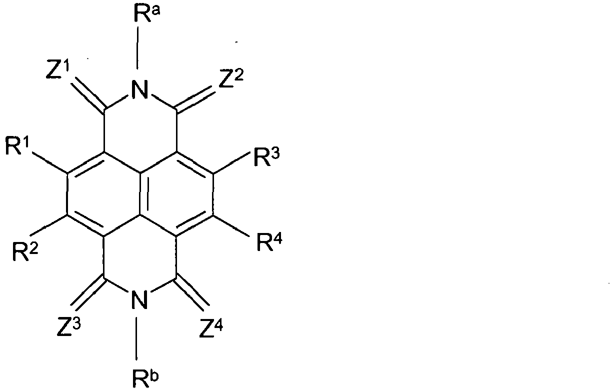 worin
R¹ und R² für Fluor und R³ und R⁴ für Wasserstoff stehen, oder
R¹ und R³ für Fluor und R² und R⁴ für Wasserstoff stehen, oder
R¹ und R⁴ für Fluor und R² und R³ für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
   oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen, bei dem man
   a1) Naphthalin-1,8;4,5-tetracarbonsäuredianhydrid einer Bromierung mit N,N'-Dibromisocyanursäure unter Erhalt einer Verbindung der allgemeinen Formel I.a 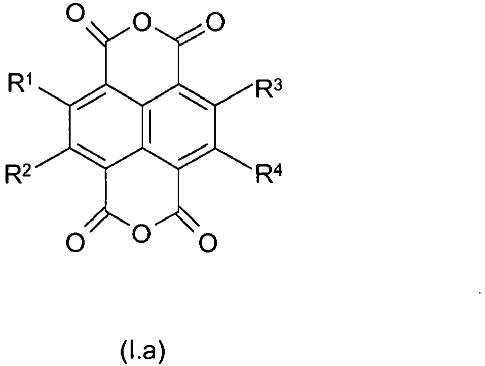 unterzieht, worin zwei oder drei oder vier der Reste R¹, R², R³ und R⁴ für Br stehen, und die übrigen Reste für Wasserstoff stehen,
   b1) die in Schritt a1) erhaltene Verbindung der Formel I.a einer Substitution des Broms durch Fluor sowie gegebenenfalls teilweise durch Wasserstoff, unterzieht,
   c1) die in Schritt b1) erhaltene Verbindung einer Umsetzung mit einem Amin der Formel R^{a}-NH₂ und gegebenenfalls einem Amin der Formel R^{b}-NH₂ unterzieht,
      oder
      die in Schritt b1) erhaltene Verbindung einer Umsetzung mit einem Amin der Formel H₂N-X-NH₂ unterzieht, wobei X für eine zweiwertige verbrückende Gruppe mit 2 bis 5 Atomen zwischen den flankierenden Bindungen steht.

Ein weiterer Gegenstand der Erfindung ist ein Verfahren zur Herstellung von Verbindungen der Formel 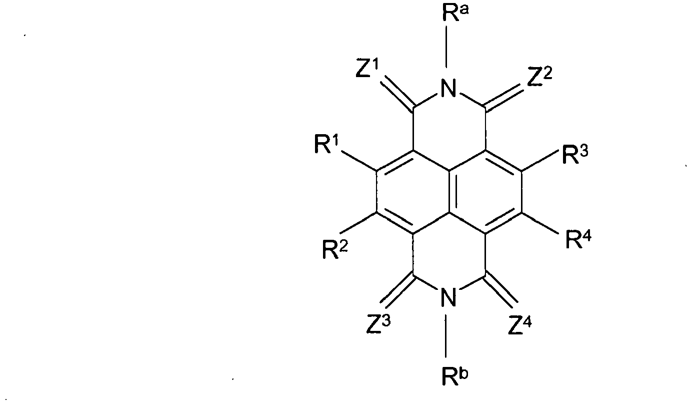 worin
R¹ und R² für Fluor und R³ und R⁴ für Wasserstoff stehen, oder
R¹ und R³ für Fluor und R² und R⁴ für Wasserstoff stehen, oder
R¹ und R⁴ für Fluor und R² und R³ für Wasserstoff stehen
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
   oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen, bei dem man
   a2) Naphthalin-1,8;4,5-tetracarbonsäuredianhydrid einer Umsetzung mit einem Amin der Formel R^{a}-NH₂ und gegebenenfalls einem Amin der Formel R^{b}-NH₂ unter Erhalt wenigstens einer Verbindung der allgemeinen Formel I.a21) 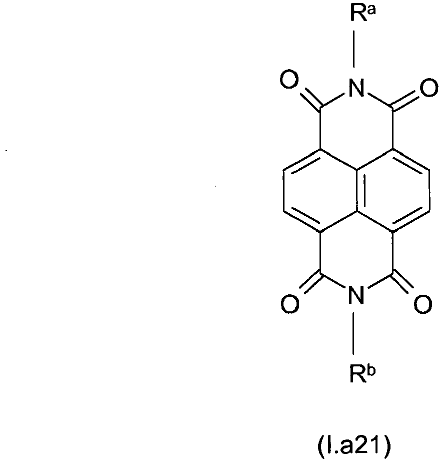 unterzieht, wobei R^{b} auch die gleiche Bedeutung wie R^{a} besitzen kann,
      oder
      Naphthalin-1,8;4,5-tetracarbonsäuredianhydrid einer Umsetzung mit einem Amin der Formel H₂N-X-NH₂ unter Erhalt wenigstens einer Verbindung der allgemeinen Formel I.a22) 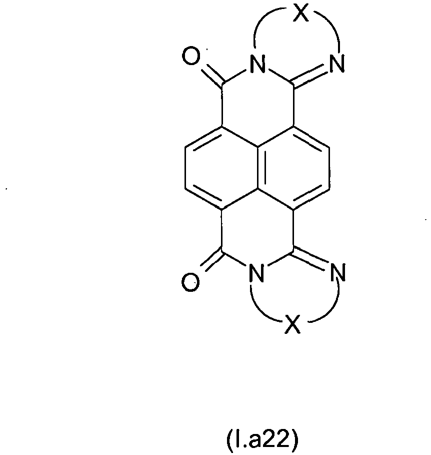 oder einem Isomeren davon unterzieht, wobei X für eine zweiwertige verbrückende Gruppe mit 2 bis 5 Atomen zwischen den flankierenden Bindungen steht.
   b2) die in Schritt a2) erhaltene(n) Verbindung(en) einer Bromierung mit N,N'-Dibromisocyanursäure unterzieht,
   c2) die in Schritt b2) erhaltene(n) Verbindung(en) einer Substitution des Broms durch Fluor sowie gegebenenfalls teilweise durch Wasserstoff, unterzieht.

### Schritt a1) und Schritt b2)

In einer ersten Ausführungsform erfolgt die Bromierung in den Schritten a1) und b2) unter Verwendung von Oleum, welches eine Konzentration von mehr als 20 % aufweist (z. B. durch Verwendung von 30%igem Oleum), als Lösungsmittel bei der Bromierung, wobei, wie zuvor beschrieben, eine tetrabromierte Verbindung erhalten wird. Zur Umsetzung wird dann vorzugsweise wenigstens 25%iges, insbesondere wenigstens 28%iges Oleum (wie z. B. 30 %iges Oleum) eingesetzt.

In einer zweiten Ausführungsform erfolgt die Bromierung in den Schritten a1) und b2) unter Verwendung von Oleum, welches eine Konzentration von höchstens 20 % aufweist. Dann werden überwiegend dibromierte Verbindungen erhalten. Das Verhältnis von Dibromisocyanursäure zu Naphthalin-1,8;4,5-tetracarbonsäuredianhydrid liegt dann vorzugsweise in einem Bereich von 1,5 : 1 bis 1 : 1, insbesondere 1,25 : 1 bis 1 : 1.

In einer bevorzugten Ausführung der zweiten Verfahrensvariante werden zur Umsetzung des Naphthalin-1,8;4,5-tetracarbonsäuredianhydrids in Schritt a2) Amine der Formel R^{a}-NH₂ und gegebenenfalls einem der Formel R^{b}-NH₂ oder Amine der Formel H₂N-X-NH₂ eingesetzt, worin R^{a}, R^{b} und X für Gruppen stehen, die sich nicht durch Umsetzung mit Dibromcyanursäure in Schritt b2) bromieren lassen. Die Reste R^{a} und R^{b} stehen dann vorzugsweise für Alkyl, Cycloalkyl, Bicycloalkyl, Heterocycloalkyl, Aryl oder Heteroaryl. Da eine zumindest teilweise Bromierung der Gruppe X jedoch in der Regel unkritisch und eventuell bei einem Einsatz der Verbindungen als Halbleiter sogar vorteilhaft sein kann, können auch bromierbare Gruppen zum Einsatz kommen, wobei die zur Bromierung eingesetzte Menge an Dibromcyanursäure in Schritt b2) dann gegebenenfalls erhöht werden muss.

### Schritt b1) und Schritt c2)

Geeignete Verfahrensbedingungen zur aromatischen nukleophilen Substitution von Bromatomen durch andere Halogenatome (Halo-Dehalogenierung), wie z. B. Fluor, sind die zuvor in Schritt ii) beschriebenen, worauf hier Bezug genommen wird.

### Schritt c1) und Schritt a2)

Wird zur Imidisierung im Schritt c1) die in Schritt b1) erhaltene Verbindung einer Umsetzung mit einem Amin der Formel R^{a}-NH₂ und gegebenenfalls einem Amin der Formel R^{b}-NH₂ unterzogen, so resultiert wenigstens eine Verbindung der allgemeinen Formel I.c1) 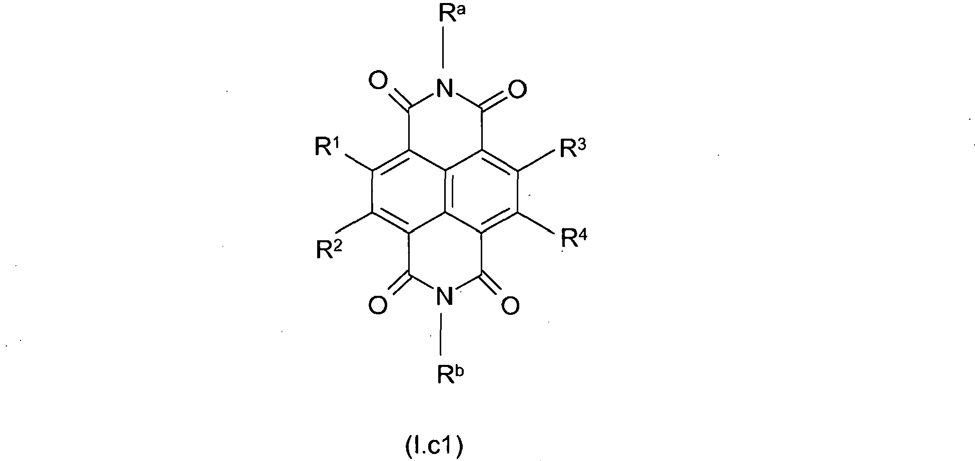 worin die Reste R¹, R², R³ und R⁴, die in der in Schritt a) erhaltenen Verbindung Ia) für Br stehen, für F stehen, wobei ein Teil der Reste R¹, R², R³ und R⁴, die in der in Schritt a) erhaltene Verbindung Ia) für Br stehen, auch für Wasserstoff stehen können,
wobei R^{b} auch die gleiche Bedeutung wie R^{a} besitzen kann (falls zur Imidisierung nur ein Amin der Formel R^{a}-NH₂ eingesetzt wird):

Wird zur Imidisierung im Schritt c1) die in Schritt b1) erhaltene Verbindung einer Umsetzung mit einem Amin der Formel H₂N-X-NH₂ unterzogen, wobei X für eine zweiwertige verbrückende Gruppe mit 2 bis 5 Atomen zwischen den flankierenden Bindungen steht, so resultiert wenigstens eine Verbindung der allgemeinen Formel I.c2) 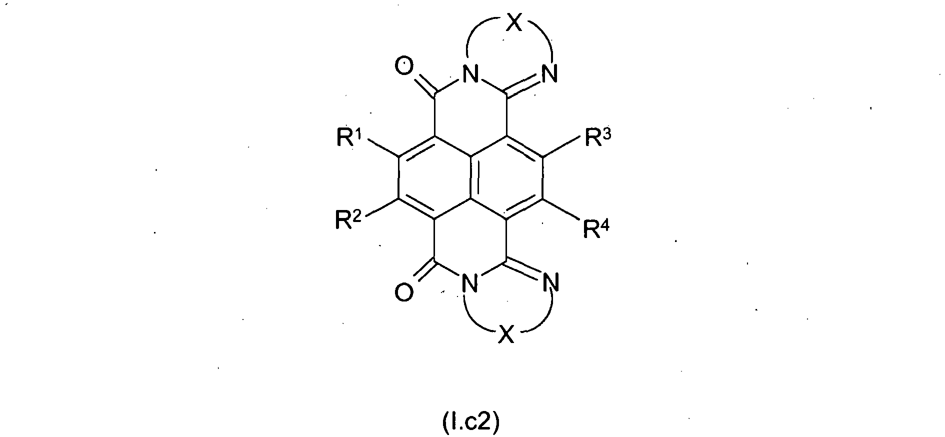 worin die Reste R¹, R², R³ und R⁴, die in der in Schritt a) erhaltenen Verbindung Ia) für Br stehen, für F stehen, wobei ein Teil der Reste R¹, R², R³ und R⁴, die in der in Schritt a) erhaltene Verbindung Ia) für Br stehen auch für Wasserstoff stehen können.

Die Imidierung der Carbonsäureanhydridgruppen in den Reaktionsschritten c1) und a2) ist prinzipiell bekannt. Vorzugsweise erfolgt die Umsetzung des Dianhydrids mit dem primären Amin in Gegenwart eines polaren aprotischen Lösungsmittels. Geeignete polare aprotische Lösungsmittel sind Stickstoffheterocyclen, wie Pyridin, Pyrimidin, Chinolin, Isochinolin, Chinaldin, N-Methylpiperidin, N-Methylpiperidon und N-Methylpyrrolidon.

Die Reaktion kann in Gegenwart eines Imidierungskatalysators vorgenommen werden. Als Imidierungskatalysatoren eignen sich organische und anorganische Säuren, z. B. Ameisensäure, Essigsäure, Propionsäure und Phosphorsäure. Geeignete Imidierungskatalysatoren sind weiterhin organische und anorganische Salze von Übergangsmetallen, wie Zink, Eisen, Kupfer und Magnesium. Dazu zählen z. B. Zinkacetat, Zinkpropionat, Zinkoxid, Eisen(II)acetat, Eisen(III)chlorid, Eisen(II)sulfat, Kupfer(II)acetat, Kupfer(II)oxid und Magnesiumacetat. Der Einsatz eines Imidierungskatalysators erfolgt vorzugsweise bei der Umsetzung aromatischer Amine und ist im Allgemeinen auch zur Umsetzung cycloaliphatischer Amine vorteilhaft. Bei der Umsetzung aliphatischer Amine, insbesondere kurzkettiger aliphatischer Amine, kann in der Regel auf den Einsatz eines Imidierungskatalysators verzichtet werden. Die Einsatzmenge des Imidierungskatalysators beträgt vorzugsweise 5 bis 80 Gew.%, besonders bevorzugt 10 bis 75 Gew.-%, bezogen auf das Gesamtgewicht der zu amidierenden Verbindung.

Vorzugsweise liegt das Molmengenverhältnis von Amin zu Dianhydrid bei etwa 2:1 bis 4:1, besonders bevorzugt 2,2:1 bis 3:1.

Die Reaktionstemperatur beträgt in den Schritten c1) bzw. a2) im Allgemeinen Umgebungstemperatur bis 200 °C, vorzugsweise 40 °C bis 180 °C. Die Umsetzung aliphatischer und cycloaliphatischer Amine erfolgt vorzugsweise in einem Temperaturbereich von etwa 60 °C bis 100 °C. Die Umsetzung aromatischer Amine erfolgt vorzugsweise in einem Temperaturbereich von etwa 120 °C bis 160 °C.

Vorzugsweise erfolgt die Umsetzung in den Reaktionsschritten c1) bzw. a2) unter einer Schutzgasatmosphäre, wie z. B. Stickstoff.

Die Reaktionsschritte c1) bzw. a2) können bei Normaldruck oder gewünschtenfalls unter erhöhtem Druck erfolgen. Ein geeigneter Druckbereich liegt im Bereich von etwa 0,8 bis 10 bar. Beim Einsatz flüchtiger Amine (Siedepunkt etwa ≤ 180 °C) arbeitet man vorzugsweise unter erhöhtem Druck.

In der Regel können die in den Reaktionsschritten c1) bzw. a2) erhaltenen Diimide ohne weitere Reinigung für die Folgeumsetzungen verwendet werden. Für einen Einsatz der Produkte als Halbleiter kann es jedoch von Vorteil sein, die Produkte einer weiteren Aufreinigung zu unterziehen. Dazu zählen beispielsweise säulenchromatographische Verfahren, wobei die Produkte vorzugsweise in einem halogenierten Kohlenwasserstoff, wie Methylenchlorid, Chlorform oder Tetrachlorethan gelöst und einer Auftrennung bzw. Filtration an Kieselgel unterzogen werden. Abschließend wird das Lösungsmittel entfernt.

Ein weiterer Gegenstand ist die Verwendung von Verbindungen der allgemeinen Formel 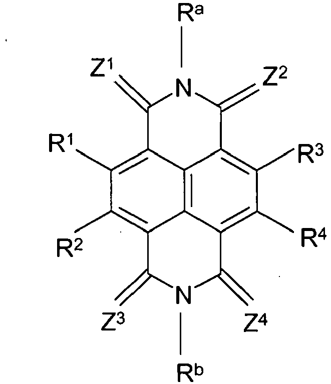 worin
wenigstens einer der Reste R¹, R², R³ und R⁴ für einen Substituenten steht, der ausgewählt ist unter Br, F und CN, und die übrigen Reste für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
   oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen,
   worin
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst,
als Halbleiter.

Erfindungsgemäß eignen sich die Verbindungen besonders vorteilhaft als organische Halbleiter. Sie fungieren dabei als n-Halbleiter und zeichnen sich durch ihre Luftstabilität aus. Weiterhin verfügen sie über eine hohe Ladungstransportmobilität und haben ein hohes on/off-Verhältnis. Sie eignen sich in besonders vorteilhafter Weise für organische Feldeffekttransistoren. Zur Herstellung von Halbleitermaterialien können die erfindungsgemäßen Verbindungen nach einem der folgenden Verfahren weiterverarbeitet werden: Drucken (Offset, Flexo, Gravur, Screen, Inkjet, Elektrofotografie), Verdampfen, Lasertransfer, Fotolithografie, Dropcasting. Sie eignen sich insbesondere für einen Einsatz in Displays und RFID-Tags.

Erfindungsgemäß eignen sich die Verbindungen weiterhin besonders vorteilhaft zur Datenspeicherung, in organischen LEDs, in der Photovoltaik, als UV-Absorber, als optischer Aufheller, für optische Label und als Fluoreszenzlabel für Biomoleküle, wie Proteine, DNA, Zucker und Kombinationen davon.

Die Erfindung wird anhand der folgenden, nicht einschränkenden Beispiele näher erläutert.

### Beispiele

### Beispiel 1 (nicht erfindungsgemäß)

### 2,3,6,7-Tetrabromnaphthalin-1,4,5,8-tetracarbonsäuredianhydrid

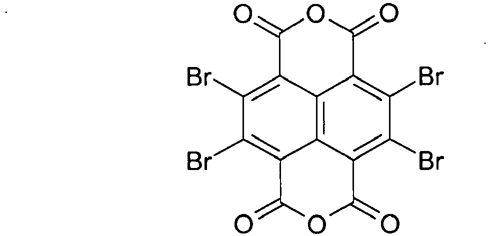

Zu einer Lösung aus 2,86 g (10 mmöl) Dibromcyanursäure in 30%igem Oleum tropft man innerhalb von vier Stunden eine Lösung von 2,68 g (10 mmol) Naphthalin-1,4-5,8-tetracarbonsäurebisanhydrid. Nach beendeter Zugabe rührt man noch eine Stunde bei Raumtemperatur weiter, bevor man das Gemisch auf 500 ml Eiswasser gießt. Der Niederschlag wird abfiltriert und mit Wasser neutral gewaschen, mit Methanol gewaschen und im Vakuum getrocknet. Man erhält 3,5 g (60 %) des 2,3,6,7-Tetrabromnaphthalin-1,4,5,8-tetracarbonsäuredianhydrids in Form eines gelblichen Feststoffes.

### Beispiel 2 (nicht erfindungsgemäß)

### Mischung aus Difluor- und Tetrafluornaphthalintetracarbonsäureanhydriden

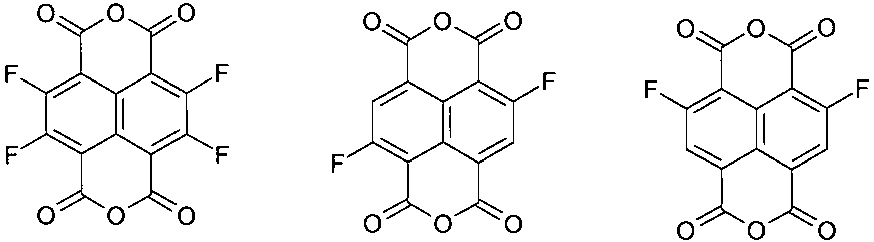

In 32 ml wasserfreies Sulfolan gibt man 5 ml Thionylchlorid, erhitzt auf 130 °C und destilliert die flüchtigen Bestandteile ab. Man kühlt auf 100 °C ab. Anschließend gibt man 0,85 g (1 mmol) der oben beschriebenen Tetrabrombisanhydridverbindung sowie 0,1g 18 Krone 6 und 1,4 g (12 mmol) getrocknetes Kaliumfluorid dazu. Man erhitzt auf 120 °C und hält das Gemisch zwei Stunden bei dieser Temperatur. Anschließend erhitzt man auf 145 °C und hält die Mischung 2 Stunden bei dieser Temperatur. Das Reaktionsgemisch wird auf Raumtemperatur abgekühlt, auf Wasser gefällt, filtriert und mit Wasser gewaschen. Gemäß massenspektroskopischer Untersuchung erhält man ein Gemisch aus Difluor- und Tetrafluornaphthalsäurebisanhydrid, welches eine Spur Trifluornapthalsäurebisanhydrid enthält.

### Beispiel 3 (nicht erfindungsgemäß)

### 2,3,6,7-Tetrabromnaphthalin-1,8;4,5-tetracarbonsäurebisanhydrid

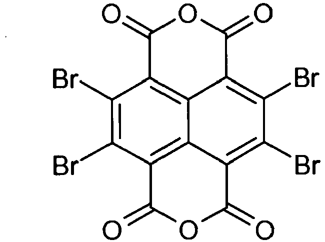

(weiteres Beispiel zur Herstellung der in Beispiel 1 genannten Verbindung)

5,36 g (20 mmol) 1,8;4,5-Naphthalintetracarbonsäurebisanhydrid werden innerhalb von einer Stunde in 100 ml 30%igem Oleum gelöst. Zu dieser Lösung gibt man bei Raumtemperatur innerhalb von vier Stunden eine Lösung von 12,6 g (44 mmol) Dibromisocyanursäure in 100 ml 30%igem Oleum. Nach beendeter Zugabe wird das Reaktionsgemisch 16 Stunden lang gerührt und anschließend vorsichtig auf 1000 ml Eiswasser gegossen, wobei ein Feststoff ausfällt. Der Rückstand wird mit verdünnter Salzsäure und wenig Methanol gewaschen und im Vakuum getrocknet. Man erhält 10,8 g (92 %) eines gelben Feststoffes.

### Beispiel 4: (nicht erfindungsgemäß)

2,6-Dibromnaphthalin-1,8;4,5-tetracarbonsäurebisanhydrid
2,7- Dibromnaphthalin-1,8;4,5-tetracarbonsäurebisanhydrid

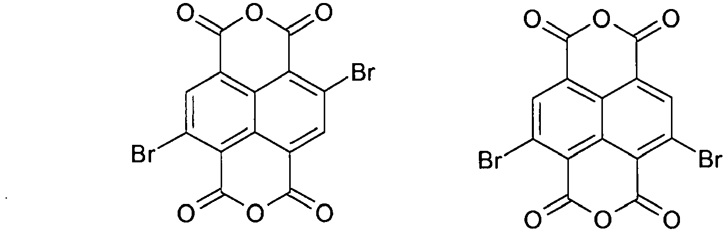

Zu einer Lösung von 2,68 g (10 mmol) 1,8;4,5-Naphthalintetracarbonsäurebisanhydrid in 50 ml 20%igen Oleum gibt man bei Raumtemperatur innerhalb von vier Stunden eine Lösung von 3,44 g (12 mmol) Dibromisocyanursäure in 100 ml 20%igen Oleum. Nach beendeter Zugabe wird noch eine Stunde gerührt bevor das Reaktionsgemisch auf 2000 ml Eiswasser gegeben wird. Das Gemisch wird 16 Stunden bei Raumtemperatur gerührt, filtriert und mit verdünnter Salzsäure und mit Methanol gewaschen und getrocknet. Man erhält 3,4 g (80 % eines gelben Feststoffes mit einem Bromwert von 36,6 % (theoretisch 37,5 %).

Gemäß ¹H-NMR in D₂SO₄ besteht das Produkt aus einem 1 : 1-Isomerengemisch der zwei oben genannten Verbindungen.

### Beispiel 5 (erfindungsgemäß verwendet)

Kondensation von ortho-Phenylendiamin mit
2,6-Dibromnaphthalin-1,8;4,5-tetracarbonsäurebisanhydrid
2,7-Dibromnaphthalin-1,8;4,5-tetracarbonsäurebisanhydrid

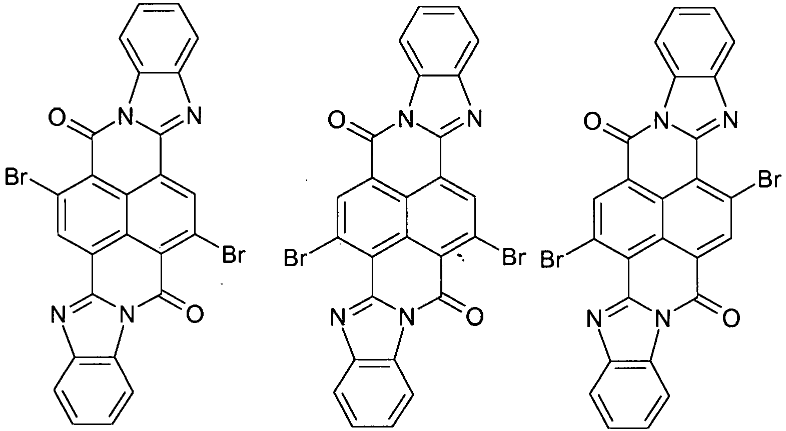

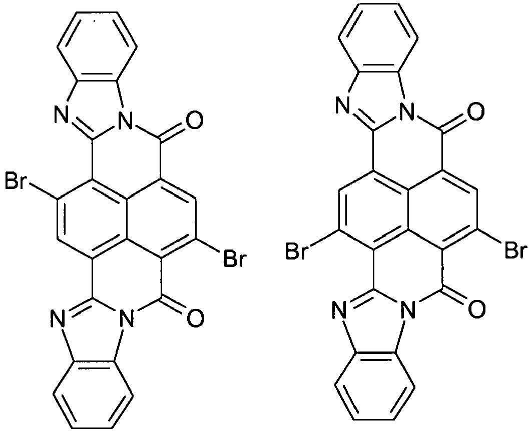

0,21 g (0,5 mmol) Dibromnaphthalintetracarbonsäurebisanhydrid werden in einem Gemisch aus 2,5 g Phenol, 0,12 g (1,1 mmol) ortho-Phenylendiamin und 0,09 g (11 mmol) Pyrazin auf 80 °C erhitzt. Das Reaktionsgemisch wird vier Stunden bei dieser Temperatur gehalten, anschließend 2,5 ml Methanol zugesetzt, auf Raumtemperatur abgekühlt und filtriert. Der Rückstand wird mit Methanol gewaschen und getrocknet. Man erhält 0,19 g (67 %) eines blau-schwarzen Feststoffes.

### Beispiel 6 (erfindungsgemäß verwendet)

Kondensation von 1,8-Diaminonaphthalin mit
2,6-Dibromnaphthalin-1,8;4,5-tetracarbonsäurebisanhydrid
2,7-Dibromnaphthalin-1,8;4,5-tetracarbonsäurebisanhydrid

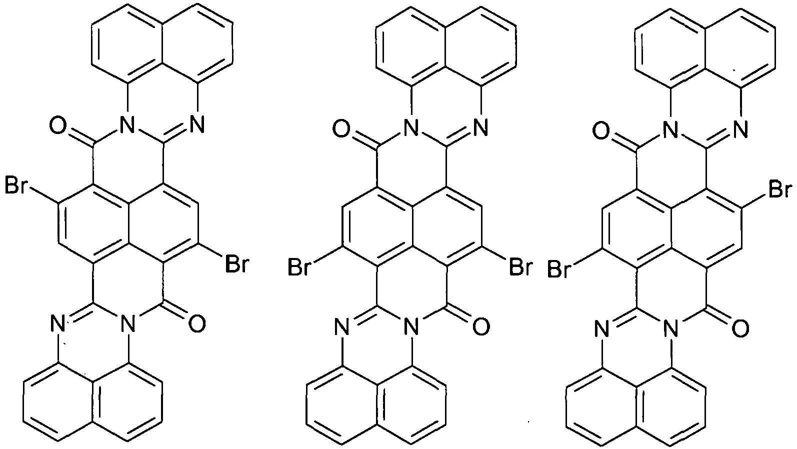

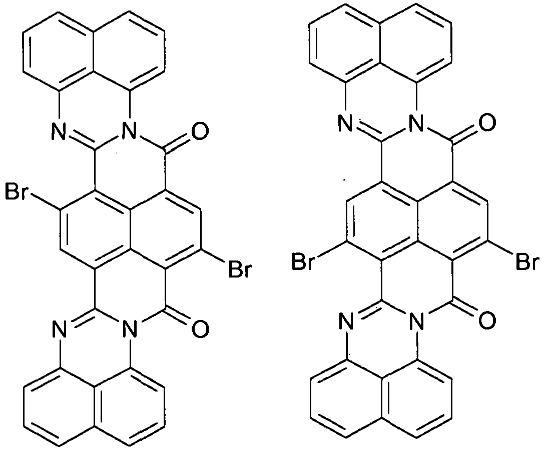

Eine Mischung aus 130 g Phenol, 11,0 g (26 mmol) Dibromnaphthalintetracarbonsäurebisanhydrid , 9,36 g (57 mmol) 1,8-Diaminonapthalin und 4,68 g (57 mmol) Pyrazin werden vier Stunden auf 80 °C erhitzt. Nach Abkühlen auf Raumtemperatur werden 130 ml Methanol zugesetzt, weitere 16 Stunden gerührt und das Reaktionsgemisch abfiltriert. Der blaue schwarze Rückstand wird mit Methanol und anschließend mit warmem Wasser gewaschen und im Vakuumtrockenschrank getrocknet.

### Beispiel 7 (nicht erfindungsgemäß)

2,3,6,7-Tetracyanonaphthalin-1,8;4,5-tetracarbonsäurebisanhydrid

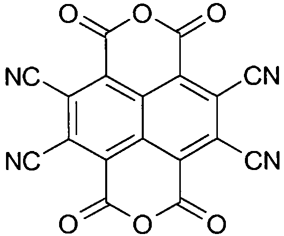

Eine Mischung von 0,58 (1 mmol) 2,3,6,7-Tetrabromnaphthalin-1,8;4,5-tetracarbonsäuredianhydrid in 50 ml Dioxan wird mit 1,76 g (15 mmol) Zinkcyanid, 70 mg (0,143 mmol) 1,1'-Bis(diphenylphosphinoferrocen) und 79 mg (143 mmol) Tris(dibenzylidenaceton)dipalladium versetzt. Das Gemisch wird 22 Stunden bei 100 °C gerührt. Man gibt 5 ml Sulfolan zu und rührt weitere 97 Stunden bei Rückfluss. Anschließend wird das Reaktionsgemisch mit Wasser verdünnt und der sich bildende Rückstand wird abfiltriert, mit Wasser gewaschen und getrocknet. Man erhält 0,46 g eines Feststoffes.

### Beispiel 8 (nicht erfindungsgemäß)

N,N'-Bis(phenethyl)-2,6-dibromnaphthalin-1,8;4,5-tetracarbonsäurediimid
N,N'-Bis(phenethyl)-2,7-dibromnaphthalin-1,8;4,5-tetracarbonsäurediimid
durch Imidierung von 2,3,6,7-Tetrabromonaphthalin-1,4;5,8-tetracarbonsäurebisanhydrid

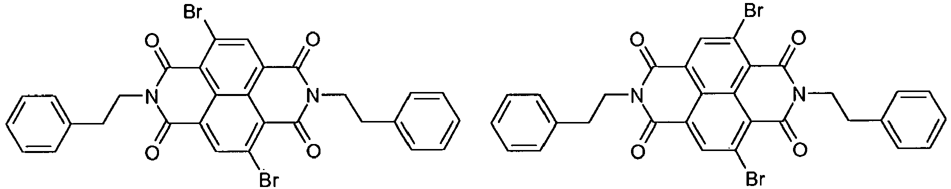

Eine Mischung aus 25 ml Xylol, 2,3 g (4 mmol) 2,3,6,7-Tetrabromnaphthalin-1,8;4,5-tetracarbonsäurebisanhydrid, 1,94 g (16 mmol) Phenethylamin wird für 6 Stunden auf 85 °C und anschließend eine Stunde auf 110 °C erhitzt. Das Reaktionsgemisch wird auf Raumtemperatur abgekühlt, filtriert und der Rückstand mit Ethanol gewaschen. Man erhält 2,3 g eines festen Produktes.

### Beispiel 9 (nicht erfindungsgemäß)

### N,N'-Di-(2,6-diisopropylphenyl)-2,3,6,7-tetrabromnaphthalin-1,8;4,5-tetracarbonsäurediimid

Naphthalin-1,8;4,5-tetracarbonsäure (200 mg, 0,343 mmol) und 2,6-Diisopropylanilin (425 mg, 2,40 mmol) werden in konzentrierter Essigsäure (5 ml) vorgelegt und 6 h bei 120 °C gerührt. Danach wird Wasser (50 ml) zugegeben, mit gesättigter Natriumhydrogencarbonat-Lösung neutralisiert und mit Chloroform extrahiert. Die vereinigten organischen Phasen werden über Natriumsulfat getrocknet und vom Lösungsmittel befreit. Der Rückstand wird mit Pentan/Dichlormethan = 2/1 an Kieselgel eluiert. Die Zielverbindung wird als die zweite, blassgelbe Fraktion isoliert. Sie kann mit präparativer HPLC (RP18) mit Dichlormethan/Methanol = 16/84 in reiner Form erhalten werden.

Ausbeute: 51 mg (0,051 mmol, 15 %)

ESI-MS: ber. für C₅₀H₅₃Br₃N₃O₄ [*M*+*H*]⁺: 1000.1558, gef.: 1000.1478; ber. für C₅₀H₅₂Br₃NaN₃O₄ [*M*+*Na*]⁺: 1022.1378, gef.: 1022.1303.

Schmp.: 344,5-346 °C

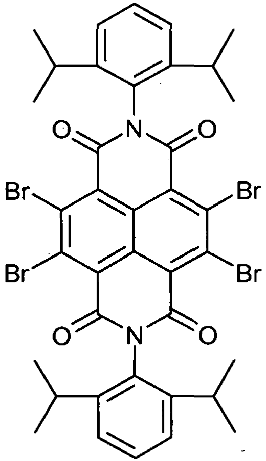

### Beispiel 10 (erfindungsgemäß verwendet)

### 2,3,6,7-Tetrabromnaphthalin-1,8;4,5-tetracarbonsäurediimid

Naphthalin-1,4,5,8-tetracarbonsäure (500 mg, 0,857 mmol) und Ammoniumacetat (1,32 g, 17,1 mmol) werden mit konzentrierter Essigsäure auf Rückfluss erhitzt, wobei zu Beginn eine gelbe Lösung entsteht. Im weiteren Verlauf fällt ein oranger Feststoff aus, der nach 2 h heiß abfiltriert wird. Die Substanz wird mit konzentrierter Essigsäure (3 ml), Wasser (5 ml), ges. Natriumhydrogencarbonat-Lösung (3 ml) und nochmals Wasser (5 ml) gewaschen und anschließend über Phosphorpentoxid getrocknet.

Ausbeute: 150 mg (0,258 mmol, 30 %)
EI-MS: ber. für C₁₄H₂Br₄N₂O₄ [*M*]⁺: 581,7, gef.: 581,7.
Schmp.: > 350 °C

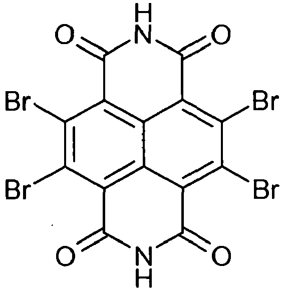

## Patentansprüche

1. Verbindungen der allgemeinen Formel 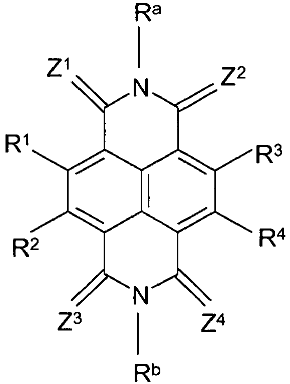 wobei
R¹ und R² für Fluor und R³ und R⁴ für Wasserstoff stehen, oder
R¹ und R³ für Fluor und R² und R⁴ für Wasserstoff stehen, oder
R¹ und R⁴ für Fluor und R² und R³ für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen,
worin
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst.

2. Verfahren zur Herstellung von Verbindungen der Formel 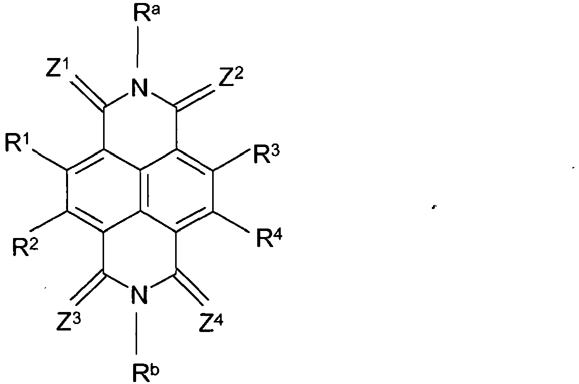 worin
R¹ und R² für Fluor und R³ und R⁴ für Wasserstoff stehen, oder
R¹ und R³ für Fluor und R² und R⁴ für Wasserstoff stehen, oder
R¹ und R⁴ für Fluor und R² und R³ für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen, bei dem man
a1) Naphthalin-1,8;4,5-tetracarbonsäuredianhydrid einer Bromierung mit N,N'-Dibromisocyanursäure unter Erhalt einer Verbindung der allgemeinen Formel I.a 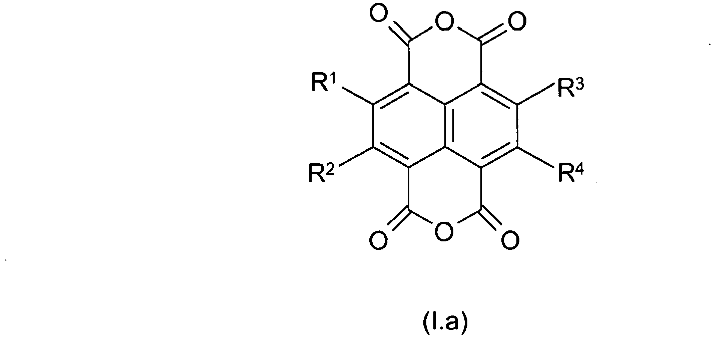 unterzieht, worin zwei oder drei oder vier der Reste R¹, R², R³ und R⁴ für Br stehen, und die übrigen Reste für Wasserstoff stehen,
b1) die in Schritt a1) erhaltene Verbindung der Formel I.a einer Substitution des Broms durch Fluor, sowie gegebenenfalls teilweise durch Wasserstoff, unterzieht,
c1) die in Schritt b1) erhaltene Verbindung einer Umsetzung mit einem Amin der Formel R^{a}-NH₂ und gegebenenfalls einem Amin der Formel R^{b}-NH₂ unterzieht,
oder
die in Schritt b1) erhaltene Verbindung einer Umsetzung mit einem Amin der Formel H₂N-X-NH₂ unterzieht, wobei X für eine zweiwertige verbrückende Gruppe mit 2 bis 5 Atomen zwischen den flankierenden Bindungen steht,
wobei
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst.

3. Verfahren zur Herstellung von Verbindungen der Formel 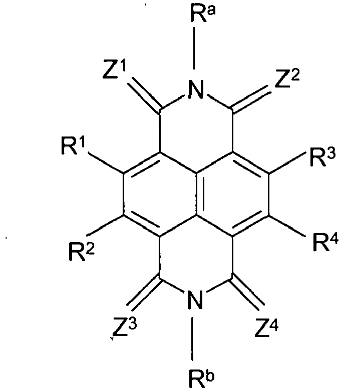 worin
R¹ und R² für Fluor und R³ und R⁴ für Wasserstoff stehen, oder
R¹ und R³ für Fluor und R² und R⁴ für Wasserstoff stehen, oder
R¹ und R⁴ für Fluor und R² und R³ für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen, bei dem man
a2) Naphthalin-1,8;4,5-tetracarbonsäuredianhydrid einer Umsetzung mit einem Amin der Formel R^{a}-NH₂ und gegebenenfalls einem Amin der Formel R^{b}-NH₂ unter Erhalt wenigstens einer Verbindung der allgemeinen Formel I.a21) 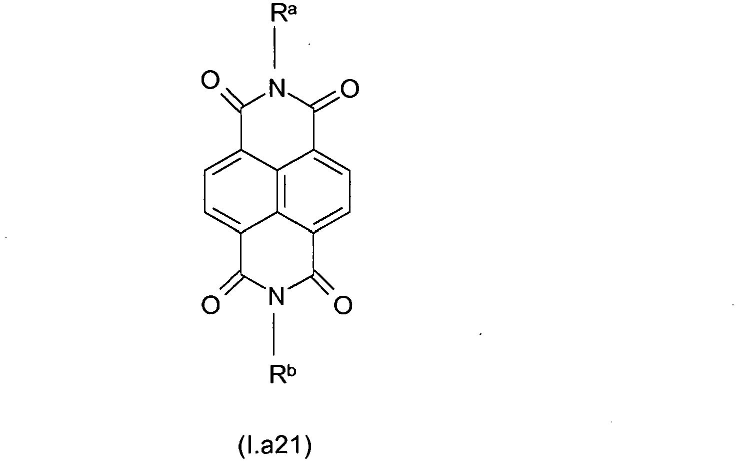 unterzieht, wobei R^{b} auch die gleiche Bedeutung wie R^{a} besitzen kann,
oder
Naphthalin-1,8;4,5-tetracarbonsäuredianhydrid einer Umsetzung mit einem Amin der Formel H₂N-X-NH₂ unter Erhalt wenigstens einer Verbindung der allgemeinen Formel I.a22) 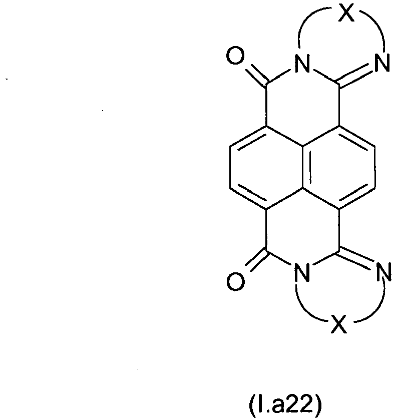 oder einem Isomeren davon unterzieht, wobei X für eine zweiwertige verbrückende Gruppe mit 2 bis 5 Atomen zwischen den flankierenden Bindungen steht.
b2) die in Schritt a2) erhaltene(n) Verbindung(en) einer Bromierung mit N,N'-Dibromisocyanursäure unterzieht,
c2) die in Schritt b2) erhaltene(n) Verbindung(en) einer Substitution des Broms durch Fluor, sowie gegebenenfalls teilweise durch Wasserstoff, unterzieht,
wobei
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst.

4. Verwendung einer Verbindung der allgemeinen Formel 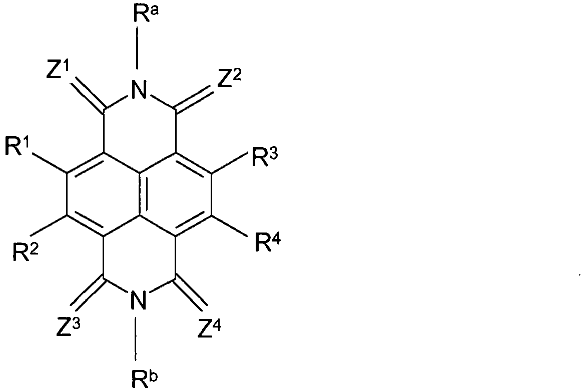 worin
wenigstens einer der Reste R¹, R², R³ und R⁴ für einen Substituenten steht, der ausgewählt ist unter Br, F und CN, und die übrigen Reste für Wasserstoff stehen,
Z¹, Z², Z³ und Z⁴ für O stehen,
R^{a} und R^{b} unabhängig voneinander für Wasserstoff oder für unsubstituiertes Alkyl, Alkenyl, Alkadienyl, Alkinyl, Cycloalkyl, Bicycloalkyl, Cycloalkenyl, Heterocycloalkyl, Aryl oder Heteroaryl stehen,
oder
einer der Reste Z¹ oder Z² für NR^{c} steht und R^{a} und R^{c} gemeinsam für eine verbrückende Gruppe mit 2 bis 5 Atomen zwischen den flankierenden Bindungen stehen und/oder einer der Reste Z³ oder Z⁴ für NR^{d} steht und R^{b} und R^{d} gemeinsam für eine verbrückende Gruppe mit 2 bis 5 Atomen zwischen den flankierenden Bindungen stehen,
als Halbleiter, insbesondere für organische Feldeffekttransistoren und die organische Photovoltaik,
worin
der Ausdruck Alkenyl geradkettige oder verzweigte Alkenylgruppen umfasst, die in Abhängigkeit von der Kettenlänge eine oder mehrere Doppelbindungen tragen können,
der Ausdruck Alkinyl Alkinylgruppen umfasst, die eine oder mehrere nicht benachbarte Dreifachbindungen aufweisen,
der Ausdruck Cycloalkenyl monocyclische, einfach ungesättigte Kohlenwasserstoffgruppen mit 3 bis 8 Kohlenstoffringgliedern umfasst,
der Ausdruck Bicycloalkyl bicyclische Kohlenwasserstoffreste mit 5 bis 10 C-Atomen umfasst,
der Ausdruck Aryl ein- oder mehrkernige aromatische Kohlenwasserstoffreste umfasst,
der Ausdruck Heterocycloalkyl nicht-aromatische, ungesättigte oder vollständig gesättigte, cycloaliphatische Gruppen mit 5 bis 8 Ringatomen, in denen 1, 2 oder 3 der Ringkohlenstoffatome durch Heteroatome, ausgewählt unter Sauerstoff, Stickstoff, Schwefel und einer Gruppe -NR^{e}- ersetzt sind, umfasst,
der Ausdruck Heteroaryl heteroaromatische, ein- oder mehrkernige Gruppen umfasst..

5. Verwendung einer Verbindung nach Anspruch 4, wobei R¹, R², R³ und R⁴ alle für Brom oder alle für Fluor oder alle für Cyano stehen..

6. Verwendung einer Verbindung nach Anspruch 4 , wobei R¹ und R² unabhängig voneinander ausgewählt sind unter Fluor und Cyano und R³ und R⁴ für Wasserstoff stehen.

7. Verwendung einer Verbindung nach Anspruch 4, wobei R¹ und R³ unabhängig voneinander ausgewählt sind unter Fluor und Cyano und R² und R⁴ für Wasserstoff stehen.

8. Verwendung einer Verbindung nach Anspruch 4, wobei R¹ und R⁴ unabhängig voneinander ausgewählt sind unter Fluor und Cyano und R² und R³ für Wasserstoff stehen.

## Claims

1. A compound of the general formula 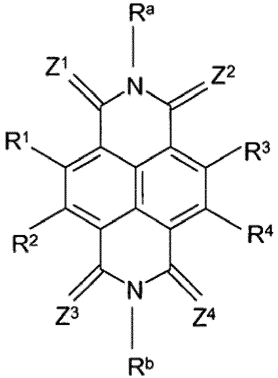 where
R¹ and R² represent fluorine and R³ and R⁴ represent hydrogen, or
R¹ and R³ represent fluorine and R² and R⁴ represent hydrogen, or
R¹ and R⁴ represent fluorine and R² and R³ represent hydrogen,
Z¹, Z², Z³ and Z⁴ represent 0,
R^{a} and R^{b} independently of one another represent hydrogen or unsubstituted alkyl, alkenyl, alkadienyl, alkynyl, cycloalkyl, bicycloalkyl, cycloalkenyl, heterocycloalkyl, aryl or heteroaryl,
or
one of the radicals Z¹ or Z² represents NR^{c} and R^{a} and R^{c} together represent a bridging group comprising 2 to 5 atoms between the flanking bonds and/or one of the radicals Z³ or Z⁴ represents NR^{d} and R^{b} and R^{b} together represent a bridging group comprising 2 to 5 atoms between the flanking bonds,
wherein
the term alkenyl encompasses straight-chain or branched alkenyl groups which, depending on the chain length, may bear one or more double bonds,
the term alkynyl encompasses alkynyl groups comprising one or more nonadjacent triple bonds,
the term cycloalkenyl encompasses monocyclic monounsaturated hydrocarbon groups comprising 3 to 8 carbon ring members,
the term bicycloalkyl encompasses bicyclic hydrocarbon radicals comprising 5 to 10 C atoms,
the term aryl encompasses mono- or polycyclic aromatic hydrocarbon radicals,
the term heterocycloalkyl encompasses nonaromatic, unsaturated or fully saturated cycloaliphatic groups comprising 5 to 8 ring atoms in which 1, 2 or 3 of the ring carbon atoms have been replaced by heteroatoms selected from oxygen, nitrogen, sulfur and an -NR^{e}- group,
the term heteroaryl encompasses heteroaromatic mono- or polycyclic groups.

2. A process for producing compounds of the formula 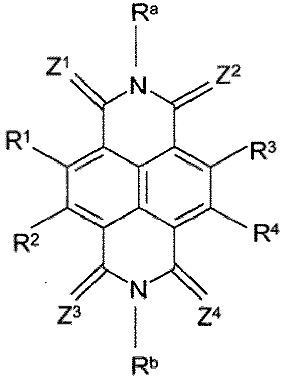 where
R¹ and R² represent fluorine and R³ and R⁴ represent hydrogen, or
R¹ and R³ represent fluorine and R² and R⁴ represent hydrogen, or
R¹ and R⁴ represent fluorine and R² and R³ represent hydrogen,
Z¹, Z², Z³ and Z⁴ represent 0,
R^{a} and R^{b} independently of one another represent hydrogen or unsubstituted alkyl, alkenyl, alkadienyl, alkynyl, cycloalkyl, bicycloalkyl, cycloalkenyl, heterocycloalkyl, aryl or heteroaryl,
or
one of the radicals Z¹ or Z² represents NR^{c} and R^{a} and R^{c} together represent a bridging group comprising 2 to 5 atoms between the flanking bonds and/or one of the radicals Z³ or Z⁴ represents NR^{d} and R^{b} and R^{d} together represent a bridging group comprising 2 to 5 atoms between the flanking bonds,
which comprises
a1) subjecting naphthalene-1,8;4,5-tetracarboxylic dianhydride to bromination with N,N'-dibromoisocyanuric acid to obtain a compound of general formula I.a, 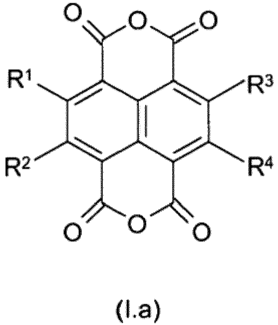 where two or three or four of the radicals R¹, R², R³ and R⁴ represent Br and the remaining radicals represent hydrogen,
b1) subjecting the compound of formula I.a obtained in step a1) to substitution of the bromine by fluorine and optionally partially by hydrogen,
c1) subjecting the compound obtained in step b1) to reaction with an amine of formula R^{a}-NH₂ and optionally an amine of formula R^{b}-NH₂,
or
subjecting the compound obtained in step b1) to reaction with an amine of formula H₂N-X-NH₂, where X represents a divalent bridging group comprising 2 to 5 atoms between the flanking bonds,
wherein
the term alkenyl encompasses straight-chain or branched alkenyl groups which, depending on the chain length, may bear one or more double bonds,
the term alkynyl encompasses alkynyl groups comprising one or more nonadjacent triple bonds,
the term cycloalkenyl encompasses monocyclic monounsaturated hydrocarbon groups comprising 3 to 8 carbon ring members,
the term bicycloalkyl encompasses bicyclic hydrocarbon radicals comprising 5 to 10 C atoms,
the term aryl encompasses mono- or polycyclic aromatic hydrocarbon radicals,
the term heterocycloalkyl encompasses nonaromatic, unsaturated or fully saturated cycloaliphatic groups comprising 5 to 8 ring atoms in which 1, 2 or 3 of the ring carbon atoms have been replaced by heteroatoms selected from oxygen, nitrogen, sulfur and an -NR^{e}- group,
the term heteroaryl encompasses heteroaromatic mono- or polycyclic groups.

3. The process for producing compounds of the formula 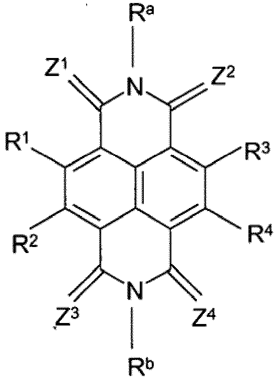 where
R¹ and R² represent fluorine and R³ and R⁴ represent hydrogen, or
R¹ and R³ represent fluorine and R² and R⁴ represent hydrogen, or
R¹ and R⁴ represent fluorine and R² and R³ represent hydrogen,
Z¹, Z², Z³ and Z⁴ represent 0,
R^{a} and R^{b} independently of one another represent hydrogen or unsubstituted alkyl, alkenyl, alkadienyl, alkynyl, cycloalkyl, bicycloalkyl, cycloalkenyl, heterocycloalkyl, aryl or heteroaryl,
or
one of the radicals Z¹ or Z² represents NR^{c} and R^{a} and R^{c} together represent a bridging group comprising 2 to 5 atoms between the flanking bonds and/or one of the radicals Z³ or Z⁴ represents NR^{d} and R^{b} and R^{b} together represent a bridging group comprising 2 to 5 atoms between the flanking bonds,
which comprises
a2) subjecting naphthalene-1,8;4,5-tetracarboxylic dianhydride to reaction with an amine of formula R^{a}-NH₂ and optionally an amine of formula R^{b}-NH₂ to obtain a compound of general formula I.a21), 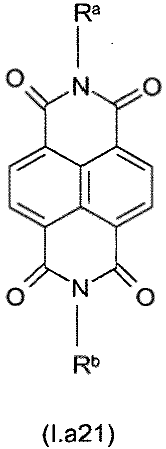 where R^{b} may also be identical to R^{a},
or
subjecting naphthalene-1,8;4,5-tetracarboxylic dianhydride to reaction with an amine of formula H₂N-X-NH₂ to obtain at least one compound of general formula I.a22) 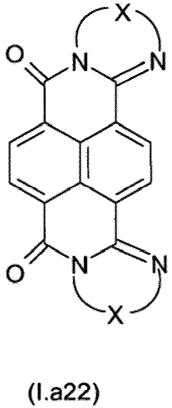 or an isomer thereof, where X is a divalent bridging group comprising 2 to 5 atoms between the flanking bonds,
b2) subjecting the compound(s) obtained in step a2) to bromination with N,N'-dibromoisocyanuric acid,
c2) subjecting the compound(s) obtained in step b2) to substitution of the bromine by fluorine and optionally partially by hydrogen,
wherein
the term alkenyl encompasses straight-chain or branched alkenyl groups which, depending on the chain length, may bear one or more double bonds,
the term alkynyl encompasses alkynyl groups comprising one or more nonadjacent triple bonds,
the term cycloalkenyl encompasses monocyclic monounsaturated hydrocarbon groups comprising 3 to 8 carbon ring members,
the term bicycloalkyl encompasses bicyclic hydrocarbon radicals comprising 5 to 10 C atoms,
the term aryl encompasses mono- or polycyclic aromatic hydrocarbon radicals,
the term heterocycloalkyl encompasses nonaromatic, unsaturated or fully saturated cycloaliphatic groups comprising 5 to 8 ring atoms in which 1, 2 or 3 of the ring carbon atoms have been replaced by heteroatoms selected from oxygen, nitrogen, sulfur and an -NR^{e}- group,
the term heteroaryl encompasses heteroaromatic mono- or polycyclic groups.

4. The use of a compound of the general formula 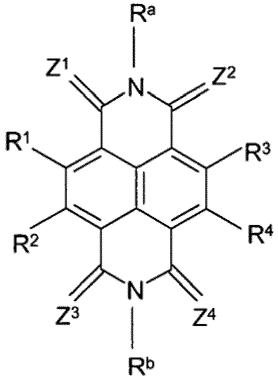 where
at least one of the radicals R¹, R², R³ and R⁴ represents a substituent selected from Br, F and CN and the remaining radicals represent hydrogen,
Z¹, Z², Z³ and Z⁴ represent 0,
R^{a} and R^{b} independently of one another represent hydrogen or unsubstituted alkyl, alkenyl, alkadienyl, alkynyl, cycloalkyl, bicycloalkyl, cycloalkenyl, heterocycloalkyl, aryl or heteroaryl,
or
one of the radicals Z¹ or Z² represents NR^{c} and R^{a} and R^{c} together represent a bridging group comprising 2 to 5 atoms between the flanking bonds and/or one of the radicals Z³ or Z⁴ represents NR^{d} and R^{b} and R^{b} together represent a bridging group comprising 2 to 5 atoms between the flanking bonds,
as a semiconductor, in particular for organic field effect transistors and organic photovoltaics,
wherein
the term alkenyl encompasses straight-chain or branched alkenyl groups which, depending on the chain length, may bear one or more double bonds,
the term alkynyl encompasses alkynyl groups comprising one or more nonadjacent triple bonds,
the term cycloalkenyl encompasses monocyclic monounsaturated hydrocarbon groups comprising 3 to 8 carbon ring members,
the term bicycloalkyl encompasses bicyclic hydrocarbon radicals comprising 5 to 10 C atoms,
the term aryl encompasses mono- or polycyclic aromatic hydrocarbon radicals,
the term heterocycloalkyl encompasses nonaromatic, unsaturated or fully saturated cycloaliphatic groups comprising 5 to 8 ring atoms in which 1, 2 or 3 of the ring carbon atoms have been replaced by heteroatoms selected from oxygen, nitrogen, sulfur and an -NR^{e}- group,
the term heteroaryl encompasses heteroaromatic mono- or polycyclic groups.

5. The use of a compound according to claim 4, wherein R¹, R², R³ and R⁴ all represent bromine, all represent fluorine or all represent cyano.

6. The use of a compound according to claim 4, wherein R¹ and R² are independently of one another selected from fluorine and cyano and R³ and R⁴ represent hydrogen.

7. The use of a compound according to claim 4, wherein R¹ and R³ are independently of one another selected from fluorine and cyano and R² and R⁴ represent hydrogen.

8. The use of a compound according to claim 4, wherein R¹ and R⁴ are independently of one another selected from fluorine and cyano and R² and R³ represent hydrogen.

## Revendications

1. Composés de formule générale 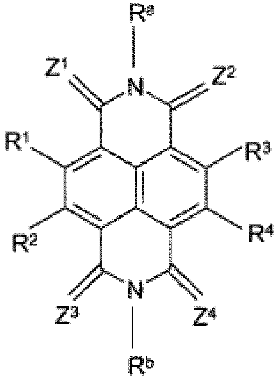 dans laquelle
R¹ et R² représentent un atome de fluor et R³ et R⁴ représentent un atome d'hydrogène, ou
R¹ et R³ représentent un atome de fluor et R² et R⁴ représentent un atome d'hydrogène, ou
R¹ et R⁴ représentent un atome de fluor et R² et R³ représentent un atome d'hydrogène,
Z¹, Z², Z³ et Z⁴ représentent 0,
R^{a} et R^{b} représentent indépendamment l'un de l'autre un atome d'hydrogène ou un groupe alkyle, alcényle, alcadiényle, alcynyle, cycloalkyle, bicycloalkyle, cycloalcényle, hétérocycloalkyle, aryle ou hétéroaryle, non substitué,
ou
l'un des radicaux Z¹ ou Z² représente NR^{c} et R^{a} et R^{c} représentent ensemble un groupe pontant ayant de 2 à 5 atomes entre les liaisons encadrantes et/ou l'un des radicaux Z³ ou Z⁴ représente NR^{d} et R^{b} et R^{d} représentent ensemble un groupe pontant ayant de 2 à 5 atomes entre les liaisons encadrantes,
où
l'expression alcényle comprend des groupes alcényle à chaîne droite ou ramifiés qui peuvent, en fonction de la longueur de chaîne, comporter une ou plusieurs doubles liaisons,
l'expression alcynyle comprend des groupes alcynyle qui comportent une ou plusieurs triples liaisons non adjacentes,
l'expression cycloalcényle comprend des groupes hydrocarbonés monocycliques mono-insaturés ayant de 3 à 8 chaînons consistant en des atomes de carbone formant le cycle,
l'expression bicycloalkyle comprend des radicaux hydrocarbonés bicycliques ayant de 5 à 10 atomes de carbone,
l'expression aryle comprend des radicaux hydrocarbonés aromatiques mono- ou polynucléaires,
l'expression hétérocycloalkyle comprend des groupes cycloaliphatiques non aromatiques, insaturés ou totalement saturés, ayant de 5 à 8 atomes formant le cycle, dans lesquels 1, 2 ou 3 des atomes de carbone formant le cycle sont remplacés par des hétéroatomes choisis parmi les atomes d'oxygène, d'azote, de soufre et un groupe -NR^{e}-,
l'expression hétéroaryle comprend des groupes hétéroaromatiques mono- ou polynucléaires.

2. Procédé pour la préparation de composés de formule 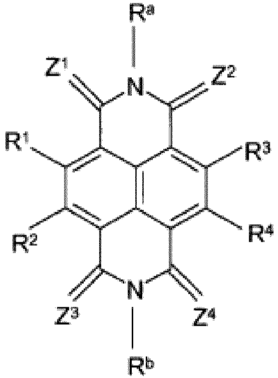 dans laquelle
R¹ et R² représentent un atome de fluor et R³ et R⁴ représentent un atome d'hydrogène, ou
R¹ et R³ représentent un atome de fluor et R² et R⁴ représentent un atome d'hydrogène, ou
R¹ et R⁴ représentent un atome de fluor et R² et R³ représentent un atome d'hydrogène,
Z¹, Z², Z³ et Z⁴ représentent 0,
R³ et R^{b} représentent indépendamment l'un de l'autre un atome d'hydrogène ou un groupe alkyle, alcényle, alcadiényle, alcynyle, cycloalkyle, bicycloalkyle, cycloalcényle, hétérocycloalkyle, aryle ou hétéroaryle, non substitué,
ou
l'un des radicaux Z¹ ou Z² représente NR^{c} et R³ et R² représentent ensemble un groupe pontant ayant de 2 à 5 atomes entre les liaisons encadrantes et/ou l'un des radicaux Z³ ou Z⁴ représente NR^{d} et R^{b} et R^{d} représentent ensemble un groupe pontant ayant de 2 à 5 atomes entre les liaisons encadrantes, dans lequel
a1) on soumet un dianhydride d'acide naphtalène-1,8;4,5-tétracarboxylique à une bromation avec de l'acide N,N'-dibromo-isocyanurique, pour obtenir un composé de formule générale I.a 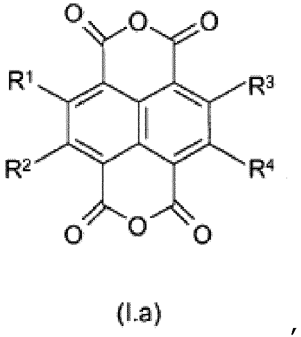 dans laquelle deux ou trois ou quatre des radicaux R¹, R², R³ et R⁴ représentent Br, et les autres radicaux représentent un atome d'hydrogène,
b1) on soumet le composé de formule I.a, obtenu dans l'étape a1), à un remplacement du brome par le fluor, ainsi qu'éventuellement en partie par l'hydrogène,
c1) on soumet le composé obtenu dans l'étape b1) à une réaction avec une amine de formule R^{a}-NH₂ et éventuellement une amine de formule R^{b}-NH₂,
ou
on soumet le composé obtenu dans l'étape b1) à une réaction avec une amine de formule H₂N-X-NH₂, X représentant un groupe pontant divalent ayant de 2 à 5 atomes entre les liaisons encadrantes,
l'expression alcényle comprenant des groupes alcényle à chaîne droite ou ramifiés qui peuvent, en fonction de la longueur de chaîne, comporter une ou plusieurs doubles liaisons,
l'expression alcynyle comprenant des groupes alcynyle qui comportent une ou plusieurs triples liaisons non adjacentes,
l'expression cycloalcényle comprenant des groupes hydrocarbonés monocycliques mono-insaturés ayant de 3 à 8 chaînons consistant en des atomes de carbone formant le cycle,
l'expression bicycloalkyle comprenant des radicaux hydrocarbonés bicycliques ayant de 5 à 10 atomes de carbone,
l'expression aryle comprenant des radicaux hydrocarbonés aromatiques mono- ou polynucléaires,
l'expression hétérocycloalkyle comprenant des groupes cycloaliphatiques non aromatiques, insaturés ou totalement saturés, ayant de 5 à 8 atomes formant le cycle, dans lesquels 1, 2 ou 3 des atomes de carbone formant le cycle sont remplacés par des hétéroatomes choisis parmi les atomes d'oxygène, d'azote, de soufre et un groupe -NR^{e}-,
l'expression hétéroaryle comprenant des groupes hétéroaromatiques mono- ou polynucléaires.

3. Procédé pour la préparation de composés de formule 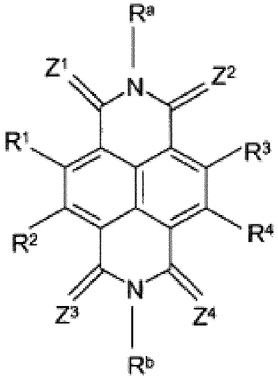 dans laquelle
R¹ et R² représentent un atome de fluor et R³ et R⁴ représentent un atome d'hydrogène, ou
R¹ et R³ représentent un atome de fluor et R² et R⁴ représentent un atome d'hydrogène, ou
R¹ et R⁴ représentent un atome de fluor et R² et R³ représentent un atome d'hydrogène,
Z¹, Z², Z³ et Z⁴ représentent 0,
R³ et R^{b} représentent indépendamment l'un de l'autre un atome d'hydrogène ou un groupe alkyle, alcényle, alcadiényle, alcynyle, cycloalkyle, bicycloalkyle, cycloalcényle, hétérocycloalkyle, aryle ou hétéroaryle, non substitué,
ou
l'un des radicaux Z¹ ou Z² représente NR^{c} et R³ et R^{c} représentent ensemble un groupe pontant ayant de 2 à 5 atomes entre les liaisons encadrantes et/ou l'un des radicaux Z³ ou Z⁴ représente NR^{d} et R^{b} et R^{d} représentent ensemble un groupe pontant ayant de 2 à 5 atomes entre les liaisons encadrantes, dans lequel
a2) on soumet du dianhydride d'acide naphtalène-1,8;4,5-tétracarboxylique à une réaction avec une amine de formule R^{a}-NH₂ et éventuellement une amine de formule R^{b}-NH₂, pour obtenir au moins un composé de formule générale I.a21) 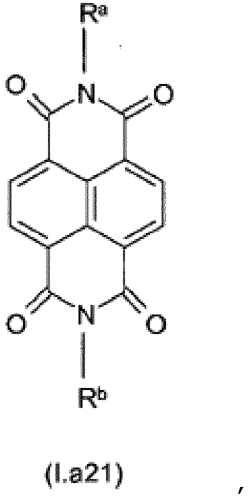 R^{b} pouvant également avoir la même signification que R^{a},
ou
on soumet un dianhydride d'acide naphtalène-1,8;4,5-tétracarboxylique à une réaction avec une amine de formule H₂N-X-NH₂, pour obtenir au moins un composé de formule générale I.a22) 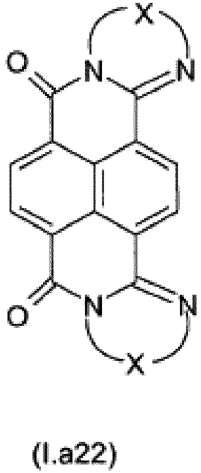 ou un isomère d'un tel composé, X représentant un groupe pontant ayant de 2 à 5 atomes entre les liaisons encadrantes,
b2) on soumet le(s) composé(s) obtenu(s) dans l'étape a2) à une bromation avec de l'acide N,N'-dibromo-isocyanurique,
c2) on soumet le(s) composé(s) obtenu(s) dans l'étape b2) à un remplacement du brome par le fluor, ainsi qu'éventuellement en partie par l'hydrogène,
l'expression alcényle comprenant des groupes alcényle à chaîne droite ou ramifiés qui peuvent, en fonction de la longueur de chaîne, comporter une ou plusieurs doubles liaisons,
l'expression alcynyle comprenant des groupes alcynyle qui comportent une ou plusieurs triples liaisons non adjacentes,
l'expression cycloalcényle comprenant des groupes hydrocarbonés monocycliques mono-insaturés ayant de 3 à 8 chaînons consistant en des atomes de carbone formant le cycle,
l'expression bicycloalkyle comprenant des radicaux hydrocarbonés bicycliques ayant de 5 à 10 atomes de carbone,
l'expression aryle comprenant des radicaux hydrocarbonés aromatiques mono- ou polynucléaires,
l'expression hétérocycloalkyle comprenant des groupes cycloaliphatiques non aromatiques, insaturés ou totalement saturés, ayant de 5 à 8 atomes formant le cycle, dans lesquels 1, 2 ou 3 des atomes de carbone formant le cycle sont remplacés par des hétéroatomes choisis parmi les atomes d'oxygène, d'azote, de soufre et un groupe -NR^{e}-,
l'expression hétéroaryle comprenant des groupes hétéroaromatiques mono- ou polynucléaires.

4. Utilisation d'un composé de formule générale 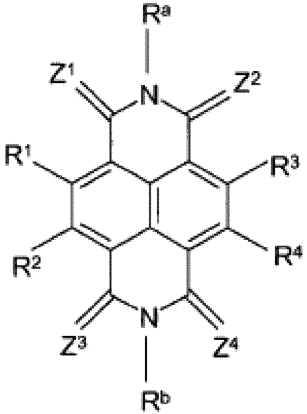 dans laquelle
au moins l'un des radicaux R¹, R², R³ et R⁴ représente un substituant qui est choisi parmi Br, F et CN, et les autres radicaux représentent un atome d'hydrogène,
Z¹, Z², Z³ et Z⁴ représentent 0,
R^{a} et R^{b} représentent indépendamment l'un de l'autre un atome d'hydrogène ou un groupe alkyle, alcényle, alcadiényle, alcynyle, cycloalkyle, bicycloalkyle, cycloalcényle, hétérocycloalkyle, aryle ou hétéroaryle, non substitué,
ou
l'un des radicaux Z¹ ou Z² représente NR^{c} et R^{a} et R^{c} représentent ensemble un groupe pontant ayant de 2 à 5 atomes entre les liaisons encadrantes et/ou l'un des radicaux Z³ ou Z⁴ représente NR^{d} et R^{b} et R^{d} représentent ensemble un groupe pontant ayant de 2 à 5 atomes entre les liaisons encadrantes,
en tant que semi-conducteur, en particulier pour des transistors organiques à effet de champ et la technique photovoltaïque,
où
l'expression alcényle comprend des groupes alcényle à chaîne droite ou ramifiés qui peuvent, en fonction de la longueur de chaîne, comporter une ou plusieurs doubles liaisons,
l'expression alcynyle comprend des groupes alcynyle qui comportent une ou plusieurs triples liaisons non adjacentes,
l'expression cycloalcényle comprend des groupes hydrocarbonés monocycliques mono-insaturés ayant de 3 à 8 chaînons consistant en des atomes de carbone formant le cycle,
l'expression bicycloalkyle comprend des radicaux hydrocarbonés bicycliques ayant de 5 à 10 atomes de carbone,
l'expression aryle comprend des radicaux hydrocarbonés aromatiques mono- ou polynucléaires,
l'expression hétérocycloalkyle comprend des groupes cycloaliphatiques non aromatiques, insaturés ou totalement saturés, ayant de 5 à 8 atomes formant le cycle, dans lesquels 1, 2 ou 3 des atomes de carbone formant le cycle sont remplacés par des hétéroatomes choisis parmi les atomes d'oxygène, d'azote, de soufre et un groupe -NR^{e}-,
l'expression hétéroaryle comprend des groupes hétéroaromatiques mono- ou polynucléaires.

5. Utilisation d'un composé selon la revendication 4, dans lequel R¹, R², R³ et R⁴ représentent tous un atome de brome ou tous un atome de fluor ou tous le groupe cyano.

6. Utilisation d'un composé selon la revendication 4, dans lequel R¹ et R² sont choisis indépendamment l'un de l'autre parmi un atome de fluor et le groupe cyano et R³ et R⁴ représentent un atome d'hydrogène.

7. Utilisation d'un composé selon la revendication 4, dans lequel R¹ et R³ sont choisis indépendamment l'un de l'autre parmi un atome de fluor et le groupe cyano et R² et R⁴ représentent un atome d'hydrogène.

8. Utilisation d'un composé selon la revendication 4, dans lequel R¹ et R⁴ sont choisis indépendamment l'un de l'autre parmi un atome de fluor et le groupe cyano et R² et R³ représentent un atome d'hydrogène.
